# EUROPEAN PATENT APPLICATION

(11) **EP 4 785 877 A1**
(43) Date of publication of application: **05.08.2026**
(21) Application number: 24884919.2
(22) Date of filing: 31.10.2024
(51) Int. Cl.: A61B 10/02

(54) **BIOPSY NEEDLE**

(30) Priority: 31.10.2023 CN 202311443250; 25.12.2023 CN 202311810938; 05.02.2024 CN 202410162641
(71) Applicant: Wuhan United Imaging Surgical Co., Ltd., Wuhan, Hubei 430206 (CN)
(72) Inventor: GU, Yafei, Wuhan, Hubei 430206 (CN); YU, Lujia, Wuhan, Hubei 430206 (CN); KE, Runkang, Wuhan, Hubei 430206 (CN); CAO, Huan, Wuhan, Hubei 430206 (CN); TAN, Bo, Wuhan, Hubei 430206 (CN)
(74) Representative: Wang, Bo
(86) International application number: PCT/CN2024/128982
(87) International publication number: WO 2025/092920

(57) **Abstract**

A biopsy needle is provided. The biopsy needle includes an inner needle cannula (120); an outer needle cannula (130), a housing (200), an inner needle cannula hub (400), an outer needle cannula hub (300), and a rotary transmission mechanism (500). The outer needle cannula (130) sleeved outside the inner needle cannula. The inner needle cannula hub (400) is movably arranged in the housing (200) and fixedly connected to the inner needle cannula (120). The outer needle cannula hub (300) is movably arranged in the housing (200) and fixedly connected to the outer needle cannula (130). The outer needle cannula hub (300) and the inner needle cannula hub (400) are capable of moving relative to each other. The rotary transmission mechanism (500) is arranged in the housing (200). The rotary transmission mechanism (500) is operable to rotate relative to the housing (200) for driving the inner needle cannula (120) and the outer needle cannula (130) to rotate synchronously.

## Description

### CROSS-REFERENCE TO RELATED APPLICATIONS

The present disclosure claims priority to Chinese Patent Application No. 202311443250.8, filed on October 31, 2023, and entitled "Biopsy Needle and Biopsy Device," the entire contents of which are incorporated herein by reference.

The present disclosure claims priority to Chinese Patent Application No. 202311810938.5, filed on December 25, 2023, and entitled "Biopsy Needle," the entire contents of which are incorporated herein by reference.

The present disclosure claims priority to Chinese Patent Application No. 202410162641.0, filed on February 5, 2024, and entitled "Biopsy Needle," the entire contents of which are incorporated herein by reference.

### TECHNICAL FIELD

The present disclosure generally relates to the field of medical devices, and in particular to a biopsy needle.

### BACKGROUND

In medical diagnosis, biopsy procedures are often performed on tumor patients to obtain part of the lesion tissue for pathological examination. Percutaneous needle biopsy refers to a procedure in which a biopsy needle is used to penetrate and excise a small amount of pathological tissue for sampling and testing under the guidance of computed tomography (CT) images, ultrasound images, or magnetic resonance imaging (MRI) images. In recent years, biopsy procedures have mainly adopted percutaneous needle biopsy due to its advantages of minimal invasiveness, less bleeding, and high success rate, which has basically replaced the traditional open surgical method.

Currently, during percutaneous needle biopsy diagnosis, a puncture device is required to cut and separate human tissue to achieve sampling and analysis. When sampling is performed using a full-core-tube biopsy needle, the process involves temporarily storing the target tissue to be cut in a chamber formed by the inner needle cannula and the core needle. Then, an elastic sheet provided on the outer needle cannula is inserted into the inner needle cannula to supplementarily cut the temporarily stored tissue, so as to obtain a target sample completely separated from the tissue, after which the needle assembly is withdrawn. In this process, the supplementary cutting by the insertion of the elastic sheet cannot guarantee complete transection. Tissue strands may remain attached, causing the sample to slip out of the needle cannula and resulting in sampling failure. Therefore, some physicians manually rotate the biopsy needle by 1 to 2 full turns to ensure effective sampling. However, manual operation presents the following drawbacks. On the one hand, for ultrasound-guided needle biopsy, physicians need to manipulate the biopsy needle with one hand, making it difficult to manually rotate the entire biopsy needle. On the other hand, manual rotation causes a large disturbance to the surrounding tissue.

### SUMMARY

Based on this, the present disclosure provides a biopsy needle.

In a first aspect, a biopsy needle is provided. The biopsy needle includes: an inner needle cannula; an outer needle cannula sleeved outside the inner needle cannula; a housing; an inner needle cannula hub movably disposed in the housing, wherein the inner needle cannula hub is fixedly connected to the inner needle cannula; an outer needle cannula hub movably disposed in the housing, wherein the outer needle cannula hub is fixedly connected to the outer needle cannula, and the outer needle cannula hub and the inner needle cannula hub are capable of moving relative to each other; and a rotary transmission mechanism disposed in the housing. The rotary transmission mechanism is operable to rotate relative to the housing for driving the inner needle cannula and the outer needle cannula to rotate synchronously.

In one or more embodiments, the rotary transmission mechanism is connected to the inner needle cannula hub and the outer needle cannula hub. After the biopsy needle completes a sampling firing operation, the inner needle cannula and the outer needle cannula are driven to rotate synchronously by controlling the rotary transmission mechanism.

In one or more embodiments, the rotary transmission mechanism includes: an actuating member rotatably connected to the housing; a transmission assembly drivingly connected to the actuating member; and a driving assembly driving the actuating member to rotate via the transmission assembly.

In one or more embodiments, the actuating member includes a rotary bracket, the rotary bracket is rotatably disposed relative to the housing, the outer needle cannula hub and the inner needle cannula hub are movable along an axial direction of the rotary bracket relative to the rotary bracket, and the rotary bracket is capable of driving the outer needle cannula hub and the inner needle cannula hub to rotate relative to the housing.

In one or more embodiments, one end of the rotary bracket along an axis of the rotary bracket is configured with an insertion key, and the insertion key is connected to at least one of the outer needle cannula hub or the inner needle cannula hub.

In one or more embodiments, the transmission assembly includes a moving member, the moving member is movably disposed relative to the housing, the moving member is drivingly connected to the rotary bracket, and the driving assembly drives the rotary bracket to rotate via the moving member.

In one or more embodiments, a peripheral side surface of the rotary bracket is provided with a driving sliding groove, and the transmission assembly further includes: a protruding portion connected to the moving member, a portion of the protruding portion being slidably disposed in the driving sliding groove. When the moving member moves relative to the housing along a first direction, the rotary bracket is driven to rotate around an axis of the housing through the protruding portion sliding along a trajectory of the driving sliding groove.

In one or more embodiments, the driving assembly includes: a first driving member. Two ends of the first driving member respectively abut against the moving member and the rotary bracket, and the first driving member is capable of providing a driving force for the moving member to move along an axial direction of the housing.

In one or more embodiments, the driving sliding groove includes a curved groove section, the moving member moves along the first direction, and the protruding portion slides along the curved groove section to drive the rotary bracket to rotate.

In one or more embodiments, the driving sliding groove includes a linear groove section extending along the axial direction of the rotary bracket, and a curved groove section extending helically around the axial direction of the rotary bracket, and two ends of the curved groove section are correspondingly connected to two ends of the linear groove section.

In one or more embodiments, each of the linear groove section and the curved groove section includes a head end and a tail end, the head end is an end facing a proximal end of the housing, and the tail end is an end facing a distal end of the housing; both a head end of the linear groove section and a tail end of the curved groove section are provided with a stop structure, or both a tail end of the linear groove section and a head end of the curved groove section are provided with the stop structure; and the stop structure is configured to prevent the protruding portion from retreating after the protruding portion passes through the stop structure from one direction.

In one or more embodiments, the stop structure at the head end of the linear groove section is a stepped structure formed by a groove bottom of the head end of the linear groove section higher than a groove bottom of the head end of the curved groove section, so as to prevent the protruding portion from retreating after the protruding portion moves from the head end of the linear groove section into the curved groove section; and the stop structure at the tail end of the curved groove section is a stepped structure formed by a groove bottom of the tail end of the curved groove section higher than the tail end of the linear groove section, so as to prevent the protruding portion from retreating after the protruding portion enters the tail end of the linear groove section from the tail end of the curved groove section. A groove bottom of the curved groove section and a groove bottom of the linear groove section are smooth surfaces.

In one or more embodiments, the protruding portion includes: a support base configured with an accommodating chamber having an opening at one end, wherein the support base is fixedly connected to the moving member; a second driving member disposed in the accommodating chamber, wherein one end of the second driving member abuts against the support base; and a movable member movably disposed in the accommodating chamber and abutting against another end of the second driving member, wherein a portion of the movable member protrudes from an end face of the support base.

In one or more embodiments, the stop structure at the head end of the linear groove section is a biasing member, the biasing member is at least partially disposed in the linear groove section; when the protruding portion moves past the biasing member along a second direction, the biasing member is biased to avoid the protruding portion; and when the protruding portion moves into the curved groove section, the biasing member resets to block the protruding portion from retreating. The first direction is opposite to the second direction.

In one or more embodiments, the driving assembly includes an elastic component, and the moving member includes: a driving screw threadedly and drivingly connected to the rotary bracket. Two ends of the elastic component respectively abut against the rotary bracket and the driving screw, such that the driving screw is movable relative to the housing.

In one or more embodiments, the moving member is provided with a first latch, the first latch is latched with the housing after the moving member moves a first preset stroke along the axial direction of the housing toward a proximal end of the housing, and the biopsy needle further includes: a first unlocking member movably connected to the housing, wherein the first unlocking member is operably connected to the first latch for operably driving the first latch to disengage from the housing.

In one or more embodiments, the biopsy needle further includes a cocking mechanism, and the cocking mechanism includes: a cocking trigger rotatably connected to the housing; and a driving rod movably disposed in the housing and drivingly connected to the cocking trigger, wherein movement of the driving rod along an axis of the housing is driven by rotation of the cocking trigger. A first stopper and a second stopper spaced apart from each other are configured on the driving rod along an extending direction of the driving rod, the first stopper is for connecting to the moving member, the second stopper is for connecting to the at least one of the outer needle cannula hub or the inner needle cannula hub, and the rotation of the cocking trigger drives the driving rod to move along the axis of the housing, thereby driving the moving member to move as well as driving the at least one of the outer needle cannula hub or the inner needle cannula hub to move.

In one or more embodiments, the moving member is provided with a stroke avoidance groove, the first stopper is disposed in the stroke avoidance groove, such that the first stopper abuts against the moving member after the driving rod moves a second preset stroke driven by the cocking trigger, thereby driving the moving member to move.

In one or more embodiments, at least one of the outer needle cannula hub or the inner needle cannula hub is provided with a second latch, and after the at least one of the outer needle cannula hub or the inner needle cannula hub moves a third preset stroke toward a proximal end of the housing, the second latch is latched with the housing.

In one or more embodiments, a locking slot is disposed on a peripheral side surface of the outer needle cannula hub or a peripheral side surface of the inner needle cannula hub, a locking protrusion is disposed on an inner wall of the housing, and when the second latch is latched with the housing, the locking protrusion is engaged in the locking slot.

In one or more embodiments, the biopsy needle further includes a core needle and a core needle seat, the core needle seat is fixedly connected to a proximal end of the housing, the core needle is fixedly connected to the core needle seat, and an end of the core needle seat facing a distal end of the housing is provided with a first guiding portion; and an end of the inner needle cannula hub or the outer needle cannula hub facing the proximal end of the housing is provided with a second guiding portion, and when the second latch is latched with the housing, the first guiding portion and the second guiding portion are engaged with each other.

In one or more embodiments, the biopsy needle further includes a shifting mechanism, and the shifting mechanism includes: a first shifting member, wherein the first shifting member is slidably connected to the insertion key for limiting a moving position of the inner needle cannula hub and a moving position of the outer needle cannula hub; a second shifting member movably disposed in the housing and rotatably connected to the first shifting member, wherein movement of the second shifting member controls a position of the first shifting member relative to the insertion key; and a gear shift button disposed on the housing and connected to the second shifting member, wherein the gear shift button is operable to move relative to the housing to control the position of the first shifting member through the second shifting member.

In one or more embodiments, the driving assembly is operable, and the driving assembly is operated after the inner needle cannula hub and the outer needle cannula hub are fired to complete sampling, so as to operably drive the rotary transmission mechanism a plurality of times, thereby enabling a plurality of synchronous rotations of the inner needle cannula hub and the outer needle cannula hub.

In one or more embodiments, the driving assembly is movably or rotatably disposed on the housing, the transmission assembly includes a gear set, and the driving assembly is drivingly connected to the rotary bracket through the gear set to drive the rotary bracket to rotate.

In one or more embodiments, the driving assembly includes a pressing assembly and a sector gear, the pressing assembly is connected to the sector gear, and the pressing assembly is pressed to drive the sector gear to rotate; the gear set is provided with a first transmission gear and a second transmission gear, the first transmission gear is drivingly connected to the sector gear, one end of the rotary bracket is provided with a third transmission gear, and the second transmission gear is drivingly connected to the third transmission gear.

In one or more embodiments, the pressing assembly includes a pressing member and an elastic member connected to each other, the pressing member is connected to the sector gear, the elastic member is configured to store elastic potential energy when the pressing member is pressed, so as to drive the pressing member to reset when the pressing member is released; and the housing is provided with an opening, and at least a portion of the pressing assembly extends to an outside of the housing through the opening.

In one or more embodiments, the rotary transmission mechanism has a preset transmission ratio, and operation of the driving assembly through the transmission assembly at least enables a rotation angle of the rotary bracket in any rotation direction to be not less than 360°.

Details of one or more embodiments of the present disclosure are set forth in the accompanying drawings and the description below. Other features, objectives, and advantages of the present disclosure will become apparent from the description, the drawings, and the claims.

### BRIEF DESCRIPTION OF THE DRAWINGS

To more clearly illustrate the technical solutions in the embodiments of the present disclosure or in the conventional technology, the accompanying drawings required for describing the embodiments or the conventional technology are briefly introduced below. Obviously, the accompanying drawings in the following description are only some embodiments of the present disclosure. For those of ordinary skill in the art, other drawings may be obtained based on the disclosed accompanying drawings without creative efforts.
FIG. 1 is a schematic diagram of an external structure of a biopsy needle according to some embodiments of the present disclosure.
FIG. 2 is a partially enlarged schematic diagram of a needle assembly in a biopsy needle according to some embodiments of the present disclosure.
FIG. 3 is a schematic diagram of an external structure of a biopsy needle from another perspective according to some embodiments of the present disclosure.
FIG. 4 is a partially enlarged schematic diagram of a biopsy needle during performing firing and sampling operations according to some embodiments of the present disclosure.
FIG. 5 is a schematic diagram of an external structure of a biopsy needle during cocking according to some embodiments of the present disclosure.
FIG. 6 is a schematic diagram of an internal structure of a biopsy needle after cocking according to some embodiments of the present disclosure.
FIG. 7 is a schematic diagram of an internal structure of a biopsy needle when not cocked according to some embodiments of the present disclosure.
FIG. 8 is a schematic diagram of a rotary transmission mechanism in a biopsy needle according to some embodiments of the present disclosure.
FIG. 9 is a cross-sectional view of a rotary transmission mechanism in a biopsy needle according to some embodiments of the present disclosure.
FIG. 10 is an exploded schematic diagram of a rotary transmission mechanism in a biopsy needle according to some embodiments of the present disclosure.
FIG. 11 is a schematic diagram of a protruding portion in a rotary transmission mechanism of a biopsy needle according to some embodiments of the present disclosure.
FIG. 12 is a schematic diagram of a protruding portion in a rotary transmission mechanism of a biopsy needle according to other embodiments of the present disclosure.
FIG. 13 is an exploded schematic diagram of a protruding portion in a rotary transmission mechanism of a biopsy needle according to other embodiments of the present disclosure.
FIG. 14 is a cross-sectional view of a rotary transmission mechanism of a biopsy needle when not cocked according to some embodiments of the present disclosure.
FIG. 15 is a schematic diagram of a rotary transmission mechanism of a biopsy needle after cocking according to some embodiments of the present disclosure.
FIG. 16 is a front view of a rotary transmission mechanism of a biopsy needle when not cocked according to some embodiments of the present disclosure.
FIG. 17 is a front view of a rotary transmission mechanism of a biopsy needle after cocking according to some embodiments of the present disclosure.
FIG. 18 is a partially enlarged schematic diagram of a rotary bracket in a biopsy needle according to some embodiments of the present disclosure.
FIG. 19 is a front view of a rotary transmission mechanism of a biopsy needle when not cocked according to other embodiments of the present disclosure.
FIG. 20 is a schematic diagram of an external structure of a protruding portion in a rotary transmission mechanism of a biopsy needle according to other embodiments of the present disclosure.
FIG. 21 is an exploded schematic diagram of a protruding portion in a rotary transmission mechanism of a biopsy needle according to other embodiments of the present disclosure.
FIG. 22 is a schematic diagram of a structure of a rotary transmission mechanism of a biopsy needle when a latch force arm is adopted to stop a protruding portion according to some embodiments of the present disclosure.
FIG. 23 is an exploded schematic diagram of a structure of a rotary transmission mechanism of a biopsy needle when a first elastic sheet is adopted to stop a protruding portion according to some embodiments of the present disclosure.
FIG. 24 is a cross-sectional schematic diagram of a structure of a rotary transmission mechanism of a biopsy needle when a first elastic sheet is adopted to stop a protruding portion according to some embodiments of the present disclosure.
FIG. 25 is a cross-sectional schematic diagram of a structure of a rotary transmission mechanism of a biopsy needle after cocking, in which a first elastic sheet is adopted to stop a protruding portion according to some embodiments of the present disclosure.
FIG. 26 is a cross-sectional schematic diagram of a structure of a rotary transmission mechanism of a biopsy needle using a first elastic sheet to stop a protruding portion when the biopsy needle is not cocked according to some embodiments of the present disclosure.
FIG. 27 is a schematic structural diagram of the cooperation among a driving rod, a rotary transmission mechanism, an inner needle cannula hub, and an outer needle cannula hub in a biopsy needle according to some embodiments of the present disclosure.
FIG. 28 is a schematic structural diagram of the cooperation between an inner needle cannula hub and an outer needle cannula hub in a biopsy needle according to some embodiments of the present disclosure.
FIG. 29 is a schematic diagram of a structure when an inner needle cannula hub and an outer needle cannula hub are mutually limited with a housing through a locking protrusion and a locking slot in a biopsy needle according to some embodiments of the present disclosure.
FIG. 30 is a schematic structural diagram of the cooperation between a rotary bracket and a housing in a biopsy needle according to some embodiments of the present disclosure.
FIG. 31 is a schematic structural diagram of the guiding and cooperating portion between an outer needle cannula hub and a core needle seat in a biopsy needle according to some embodiments of the present disclosure.
FIG. 32 is a partially enlarged structural diagram of a biopsy needle according to some embodiments of the present disclosure.
FIG. 33 is a schematic diagram of an internal structure of a biopsy needle (without completing sampling firing) according to some embodiments of the present disclosure.
FIG. 34 is a schematic diagram of an internal structure of a biopsy needle (after completion of sampling firing) according to some embodiments of the present disclosure.
FIG. 35 is a schematic diagram of an internal structure of a biopsy needle after firing according to some embodiments of the present disclosure.
FIG. 36 is a schematic diagram of a rotary transmission mechanism of a biopsy needle according to some embodiments of the present disclosure.
FIG. 37a is a schematic diagram of an external view of gear visualization of a biopsy needle according to some embodiments of the present disclosure.
FIG. 37b is a schematic diagram of an internal view of gear visualization of a biopsy needle according to some embodiments of the present disclosure.
FIG. 38a is a schematic diagram of an external view of gear visualization of a biopsy needle according to some embodiments of the present disclosure.
FIG. 38b is a schematic diagram of an internal view of gear visualization of a biopsy needle according to some embodiments of the present disclosure.
FIG. 39 is a schematic diagram of a one-touch firing structure of a biopsy needle according to some embodiments of the present disclosure.

### REFERENCE NUMERALS:

Needle assembly 100; core needle 110; inner needle cannula 120; outer needle cannula 130; second elastic sheet 131; housing 200; locking protrusion 210; annular groove 220; outer needle cannula hub 300; second latch 310; locking slot 320; inner needle cannula hub 400; guiding channel 410; rotary transmission mechanism 500; rotary bracket 510; insertion key 511; driving sliding groove 512; linear groove section 5121; first limiting step 5121a; curved groove section 5122; second limiting step 5122a; latch force arm 513; flange 514; moving member 520; accommodating groove 521; first latch 522; stroke avoidance groove 523; protruding portion 530; support base 531; second driving member 532; movable member 533; first driving member 540; first elastic sheet 550; connecting portion 551; transition portion 552; stopping portion 553; first unlocking member 600; connecting rod 610; first unlocking button 620; second unlocking button 630; cocking mechanism 700; cocking trigger 710; driving rod 720; first stopper 721; second stopper 722; guiding protrusion 723; rotating shaft 730; shifting mechanism 800; gear shift button 810; first shifting member 820; third driving member 910; core needle seat 920; guide boss 921; driving assembly 251; pressing assembly 2511; sector gear 2512; rotation axis 2513; gear set 252; first transmission gear 2521; second transmission gear 2522; transition member 253; boss 2531; stop surface 2533; transmission member 254; third transmission gear 2541; pressing member 5111; elastic member 5112; opening 201; adjusting member 260; button 261; matching portion 262; limiting hole 202; limiting portion 203; sliding groove 2532; transmission assembly 3410; driving screw 3411; cocking latch 3412; rotary firing button 4311; rotary firing latch 4312; first elastic member 4313; elastic component 3431; cocking push block 4321; cocking slider 3810; third latch 3210; stop block 3110; slot 3521; fixed ring 3700; bearing 3800; button 3830; gear position window 00; sampling firing button 401.

### DETAILED DESCRIPTION

The technical solutions in the embodiments of the present disclosure will be clearly and completely described below with reference to the accompanying drawings. Obviously, the described embodiments are merely some embodiments of the present disclosure, rather than all of the embodiments. Based on the embodiments in the present disclosure, all other embodiments obtained by a person of ordinary skill in the art without making inventive efforts shall fall within the scope of protection of the present disclosure.

In order to make the aforementioned objectives, features, and advantages of the present disclosure more obvious and easy to understand, the detailed embodiments of the present disclosure will be described in detail below with reference to the accompanying drawings. Many specific details are set forth in the following description in order to provide a thorough understanding of the present disclosure. However, the present disclosure can be implemented in many other manners different from those described herein. A person of ordinary skill in the art can make similar modifications without departing from the spirit of the present disclosure. Therefore, the present disclosure is not limited by the specific embodiments disclosed below.

In the description of the present disclosure, it should be understood that the orientations or positional relationships indicated by the terms "center," "longitudinal," "transverse," "length," "width," "thickness," "upper," "lower," "front," "rear," "left," "right," "vertical," "horizontal," "top," "bottom," "inner," "outer," "clockwise," "counterclockwise," "axial direction," "radial direction," "circumferential direction," and the like, are based on the orientations or positional relationships shown in the accompanying drawings. These terms are merely for the convenience of describing and simplifying the present disclosure, and do not indicate or imply that the indicated apparatus or elements must have a specific orientation, or be constructed and operated in a specific orientation. Therefore, these terms should not be construed as a limitation on the present disclosure.

Furthermore, the terms "first" and "second" are used merely for description purposes, and should not be construed as indicating or implying relative importance or implicitly indicating the quantity of the technical features indicated. Thus, a feature defined with "first" or "second" may explicitly or implicitly include at least one of the features. In the description of the present disclosure, "a plurality of" refers to at least two, for example, two, three, and so on, unless otherwise explicitly and specifically defined.

In the present disclosure, unless otherwise explicitly provided and defined, the terms "disposed," "connected," "connecting," "fixed," and the like should be broadly understood. For example, the connection may be a fixed connection, a detachable connection, or an integral connection; it may be a mechanical connection or an electrical connection; it may be directly connected, or indirectly connected through an intermediate medium; it may be an internal communication between two elements or an interactive relationship between two elements, unless otherwise explicitly defined. For a person of ordinary skill in the art, the specific meanings of the above terms in the present disclosure can be understood according to specific circumstances.

In the present disclosure, unless otherwise explicitly provided and defined, a first feature being "on" or "below" a second feature may mean that the first and second features are in direct contact, or the first and second features are in indirect contact through an intermediate medium. Moreover, "above," "over," and "on" a second feature for a first feature may mean that the first feature is directly above or obliquely above the second feature, or merely indicates that the horizontal height of the first feature is higher than that of the second feature. A first feature being "below," "under," and "beneath" a second feature may mean that the first feature is directly below or obliquely below the second feature, or merely indicates that the horizontal height of the first feature is lower than that of the second feature.

It should be noted that when an element is referred to as "fixed to" or "disposed on" another element, it can be directly on the other element or there may be an interposed element. When an element is considered "connected" to another element, it can be directly connected to the other element, or there may be an interposed element at the same time. The terms "vertical," "horizontal," "upper," "lower," "left," "right," and similar expressions used herein are merely for description purposes, and do not indicate the sole embodiment.

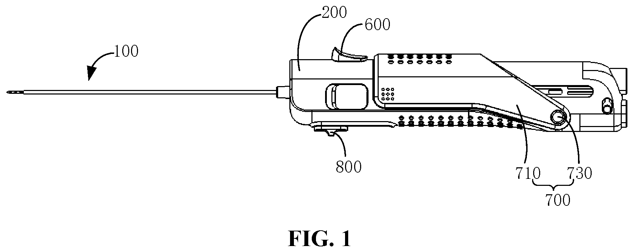

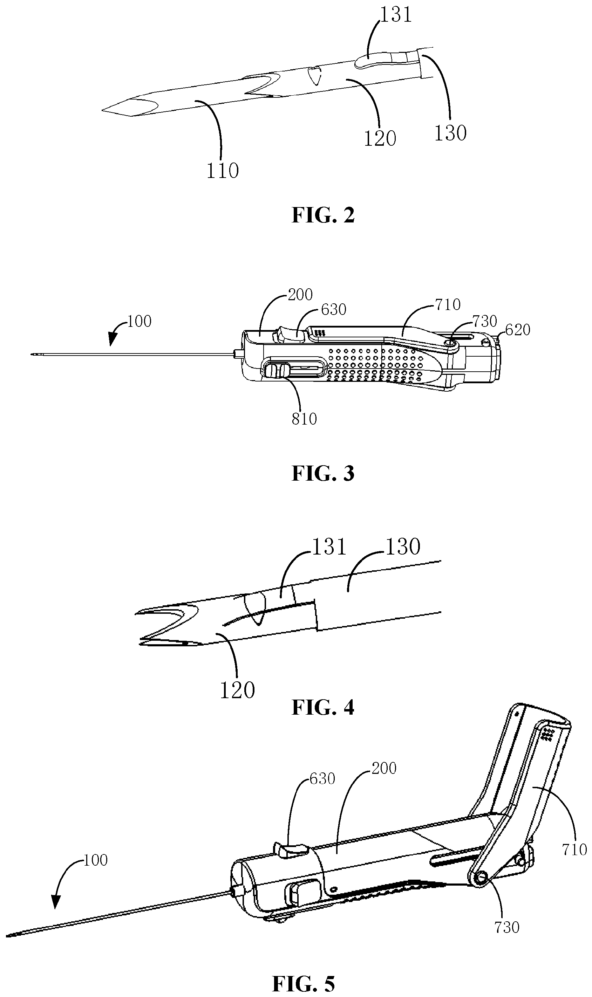

Referring to FIG. 1 to FIG. 7, an embodiment of the present disclosure provides a biopsy needle. The biopsy needle includes an inner needle cannula 120, an outer needle cannula 130 sleeved outside the inner needle cannula 120, a housing 200, an inner needle cannula hub 400 movably disposed in the housing 200, an outer needle cannula hub 300 movably disposed in the housing 200, and a rotary transmission mechanism 500, disposed in the housing 200. The inner needle cannula hub 400 is fixedly connected to the inner needle cannula 120. The outer needle cannula hub 300 is fixedly connected to the outer needle cannula 130. The outer needle cannula hub 300 and the inner needle cannula hub 400 are capable of moving relative to each other. The rotary transmission mechanism 500 is operable to rotate relative to the housing 200 for driving the inner needle cannula 120 and the outer needle cannula 130 to rotate synchronously.

The inner needle cannula 120 and the outer needle cannula 130 are configured to cut and separate a sample tissue within a sampling space, thereby achieving the purpose of sampling. The housing 200 is configured to accommodate the inner needle cannula hub 400, the outer needle cannula hub 300, and other components.

In some embodiments, the biopsy needle further includes a core needle. The housing 200 is also configured to fixedly connect to a core needle 110, so that the core needle 110 is fixed relative to the housing 200. Since the inner needle cannula 120 is sleeved on the core needle 110, the inner needle cannula hub 400 is configured to fixedly connect to the inner needle cannula 120. This enables the inner needle cannula hub 400 to move relative to the housing 200, so that the inner needle cannula 120 can move relative to the core needle 110, thereby causing a hollow area between the inner needle cannula 120 and the core needle 110 to be filled with the sample tissue.

In some embodiments, the inner needle cannula 120 is sleeved on the core needle 110, and the outer needle cannula 130 is sleeved on the inner needle cannula hub 400. The core needle 110, the inner needle cannula 120, and the outer needle cannula 130 are coaxially disposed.

In some embodiments, an elastic sheet or a cutting blade may be disposed on the outer needle cannula 130, and a hole or a slot may be disposed on the inner needle cannula 120. Since the outer needle cannula 130 is sleeved on the inner needle cannula 120, the outer needle cannula 130 is fixedly connected to the outer needle cannula hub 300. The outer needle cannula hub 300 can move relative to the housing 200, and the outer needle cannula hub 300 and the inner needle cannula hub 400 can move relative to each other, thereby enabling the outer needle cannula 130 to move relative to the inner needle cannula 120. This allows the elastic sheet (or cutting blade) on the outer needle cannula 130 to pass through a slot (or cutting hole) on the inner needle cannula 120 and enter the inner part of the inner needle cannula 120, so that the outer needle cannula 130 can cut a target tissue between the inner needle cannula 120 and the core needle 110.

In some embodiments, an elastic sheet or a cutting blade may be disposed on the inner needle cannula 120, and a hole or a slot may be disposed on the outer needle cannula 130. The relative movement between the inner needle cannula 120, the outer needle cannula 130, and the housing 200 can enable the elastic sheet (or cutting blade) on the inner needle cannula 120 to pass out from a slot (or cutting hole) on the outer needle cannula 130 and then pass into the outer needle cannula 130 from another slot, so that the elastic sheet (or cutting blade) on the inner needle cannula 120 can cut the target tissue between the inner needle cannula 120 and the outer needle cannula 130.

The embodiments of the present disclosure provide for disposing the rotary transmission mechanism 500 in the housing 200, and the rotary transmission mechanism 500 is operable to rotate relative to the housing 200. The inner needle cannula 120 and the outer needle cannula 130 are driven to rotate synchronously by the rotary transmission mechanism 500, so that the inner needle cannula 120 and the outer needle cannula 130 can rotate relative to the core needle 110 after the biopsy needle completes sampling firing. During the rotation of the inner needle cannula 120 and the outer needle cannula 130, the elastic sheet (or cutting blade) on the outer needle cannula 130 or the inner needle cannula 120 also rotates together, to perform annular cutting on the sample tissue within the inner needle cannula 120. Through the above structural form, the tissue uncut by linear cutting can be cut again by a manner of rotary cutting of the needle cannulas. This achieves the purpose of completely cutting and separating the target tissue sample from the surrounding tissue, thereby reducing the pulling of the uncut tissue during removal, which leads to some unnecessary tissue damage. Moreover, through the rotary transmission mechanism 500, synchronous in-situ rotary cutting of the inner and outer needle cannulas 130 can be manually controlled after the firing is completed. The cutting radius is small, which avoids a larger rotation radius at the needle tip caused by manual operation of the biopsy needle's overall rotation, thereby avoiding secondary injury to the surrounding important organs and the risk of enlargement of the puncture wound. This greatly improves safety and reduces the pain sensation of a patient at the same time.

In one embodiment, the biopsy needle includes a needle assembly 100, the needle assembly 100 including the inner needle cannula 120 and the outer needle cannula 130. The housing 200 has an accommodating chamber. The inner needle cannula hub 400 is disposed in the accommodating chamber, and the inner needle cannula hub 400 is capable of moving relative to the housing 200. The outer needle cannula hub 300 is disposed in the accommodating chamber, and the outer needle cannula hub 300 is capable of moving relative to the housing 200. The rotary transmission mechanism 500 is disposed in the accommodating chamber.

The needle assembly 100 is configured to cut and separate the sample tissue within the sampling space, thereby achieving the purpose of sampling.

In one embodiment, the rotary transmission mechanism 500 is connected to the inner needle cannula hub 400 and the outer needle cannula hub 300. After the biopsy needle completes sampling firing, the inner needle cannula 120 and the outer needle cannula 130 are driven to rotate synchronously by controlling the rotary transmission mechanism 500. A second elastic sheet 131 may be disposed on the outer needle cannula 130. Since the outer needle cannula 130 is sleeved on the inner needle cannula 120, the outer needle cannula 130 is fixedly connected to the outer needle cannula hub 300. The outer needle cannula hub 300 can move relative to the housing 200, and the outer needle cannula hub 300 and the inner needle cannula hub 400 can move relative to each other, thereby enabling the outer needle cannula 130 to move relative to the core needle 110 and the inner needle cannula 120. This allows the second elastic sheet 131 on the outer needle cannula 130 to pass through a slot on the inner needle cannula 120 and enter the inner part of the inner needle cannula 120, so that the outer needle cannula 130 can cut a target tissue between the inner needle cannula 120 and the core needle 110. Alternatively, when the second elastic sheet 131 is disposed on the inner needle cannula 120 and a slot is disposed on the outer needle cannula 130, the relative movement between the inner needle cannula 120, the outer needle cannula 130, and the housing 200 can enable the second elastic sheet 131 on the inner needle cannula 120 to pass out from a slot on the outer needle cannula 130 and then pass into the outer needle cannula 130 from another slot, so that the second elastic sheet 131 on the inner needle cannula 120 can cut the target tissue between the inner needle cannula 120 and the outer needle cannula 130. By disposing the rotary transmission mechanism 500 in the accommodating chamber of the housing 200, and the rotary transmission mechanism 500 is operable to rotate relative to the housing 200, after the biopsy needle completes sampling firing (for example, after the inner needle cannula hub 400 and the outer needle cannula hub 300 respectively move a preset stroke along an axis of the housing 200 to complete sampling firing), both can be connected to the rotary transmission mechanism 500. Thus, the inner needle cannula 120 and the outer needle cannula 130 are driven to rotate synchronously by the rotary transmission mechanism 500, which further enables the inner needle cannula 120 and the outer needle cannula 130 to rotate relative to the core needle 110. During the rotation of the inner needle cannula 120 and the outer needle cannula 130, the second elastic sheet 131 on the outer needle cannula 130 extending into the inner needle cannula 120 also rotates together, to perform annular cutting on the sample tissue within the inner needle cannula 120. In the present disclosure, by connecting the rotary transmission mechanism 500 to the inner needle cannula hub 400 and the outer needle cannula hub 300, the rotary transmission mechanism 500 is always connected to the inner needle cannula hub 400 and the outer needle cannula hub 300 during the firing process of the biopsy needle and after the firing process. This thereby improves the reliability of the rotary transmission mechanism 500 in driving the inner needle cannula hub 400 and the outer needle cannula hub 300 to rotate relative to the housing 200.

As shown in FIG. 1 to FIG. 5, in one embodiment, a distal end of the inner needle cannula 120 has a cutting edge structure and is capable of cutting human tissue. A slot is provided on a side wall of the inner needle cannula 120, wherein the slot penetrates the side wall of the inner needle cannula 120. A distal end of the outer needle cannula 130 is provided with a second elastic sheet 131. When the outer needle cannula 130 moves along an axis of the needle assembly 100 relative to the inner needle cannula 120, the second elastic sheet 131 may pass through the slot on the inner needle cannula 120 and enter an interior of the inner needle cannula 120, so as to cut the tissue between the inner needle cannula 120 and the core needle 110.

In other embodiments, except that the structures of the inner needle cannula 120 and the outer needle cannula 130 are different, other structures remain unchanged. Specifically, the inner needle cannula 120 is provided with the second elastic sheet 131. The outer needle cannula 130 is provided with two slots that are spaced apart. The second elastic sheet 131 passes through the slots from an inner side of the outer needle cannula 130 and rests on a tube wall between the two slots. The second elastic sheet 131 is configured as an arc-shaped structure. When the inner needle cannula 120 moves relative to the outer needle cannula 130, the second elastic sheet 131 is capable of entering an interior of the outer needle cannula 130 from the slot near the distal end of the outer needle cannula 130, so as to cut the target tissue between the inner needle cannula 120 and the outer needle cannula 130.

It is to be noted that, in the present disclosure, for the biopsy needle, the proximal end refers to an end near an operator's operating end, and the distal end refers to an end away from the operator's operating end. The distal end of each of the core needle 110, the inner needle cannula 120, and the outer needle cannula 130 refers to an end away from the operating end. The inner needle cannula hub 400 and the outer needle cannula hub 300 are disposed in an accommodating chamber of the housing 200 at an end near the proximal end. The rotary transmission mechanism 500 is disposed in the accommodating chamber of the housing 200 at an end near the distal end.

In one embodiment, the rotary transmission mechanism includes an actuating member rotatably connected to the housing 200, a transmission assembly drivingly connected to the actuating member, and a driving assembly driving the actuating member to rotate via the transmission assembly.

When the driving assembly drives the transmission assembly, the transmission assembly is capable of driving the actuating member to rotate. The actuating member can rotate relative to the housing 200, so as to drive the outer needle cannula hub 300 and the inner needle cannula hub 400 to rotate relative to the housing 200.

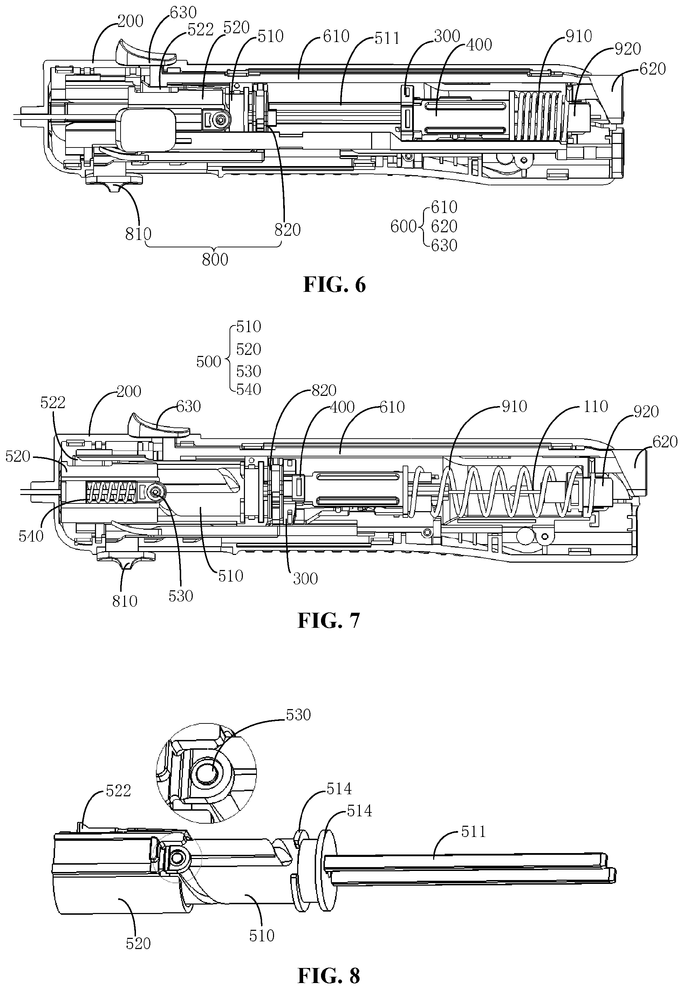

As shown in FIG. 6, FIG. 7, FIG. 9, FIG. 10, and so on, in one embodiment, the actuating member includes the rotary bracket 510. The rotary bracket 510 is rotatably disposed relative to the housing 200. The outer needle cannula hub 300 and the inner needle cannula hub 400 are movable along the axial direction of the rotary bracket 510 relative to the rotary bracket 510. The rotary bracket 510 is capable of driving the outer needle cannula hub 300 and the inner needle cannula hub 400 to rotate relative to the housing 200.

In one embodiment, one end of the rotary bracket 510 along an axis of the rotary bracket 510 is configured with an insertion key 511. The insertion key 511 is connected to the outer needle cannula hub 300 and/or the inner needle cannula hub 400.

By configuring the insertion key 511 on the rotary bracket 510, and connecting the insertion key 511 to the outer needle cannula hub 300 and/or the inner needle cannula hub 400, the connection between the rotary bracket 510 and the inner needle cannula hub 400 and the outer needle cannula hub 300 is achieved. It is to be noted that the insertion key 511 extends along the axial direction of the rotary bracket 510, and the inner needle cannula hub 400 and the outer needle cannula hub 300 are capable of moving relative to the insertion key 511. The inner needle cannula hub 400 and the outer needle cannula hub 300 are always connected to the insertion key during a cocking and firing process. This enables the inner needle cannula hub 400 and the outer needle cannula hub 300 to successfully complete the cocking and firing process. At the same time, the inner needle cannula hub 400 and the outer needle cannula hub 300 are also enabled to rotate relative to the housing 200, driven by the rotary bracket 510. Specifically, the insertion key 511 may be disposed on the rotary bracket 510 by way of integral formation, or by way of adhesive bonding.

In one embodiment, the rotary bracket 510 includes two insertion keys 511 that are spaced apart. The two insertion keys 511 are respectively inserted into slots on the outer needle cannula hub 300 and the inner needle cannula hub 400.

By configuring the insertion key 511 on the rotary bracket 510, after the outer needle cannula hub 300 and the inner needle cannula hub 400 are fired (that is, after the outer needle cannula hub 300 and the inner needle cannula hub 400 move a preset distance along the axis of the housing 200 towards the distal end of the housing 200), the two insertion keys 511 are respectively inserted into the slots on the inner needle cannula hub 400 and the outer needle cannula hub 300, so as to achieve the connection between the rotary bracket 510 and the inner needle cannula hub 400 and the outer needle cannula hub 300. Thereby, the outer needle cannula hub 300, the inner needle cannula hub 400 and the rotary bracket 510 are made relatively fixed, so as to drive the outer needle cannula hub 300 and the inner needle cannula hub 400 to rotate relative to the housing 200 when the rotary bracket 510 rotates relative to the housing 200.

In some embodiments, the two insertion keys 511 may be disposed on a proximal end of the rotary bracket 510. The two slots may be respectively disposed on the distal end of the outer needle cannula hub 300 and the distal end of the inner needle cannula hub 400.

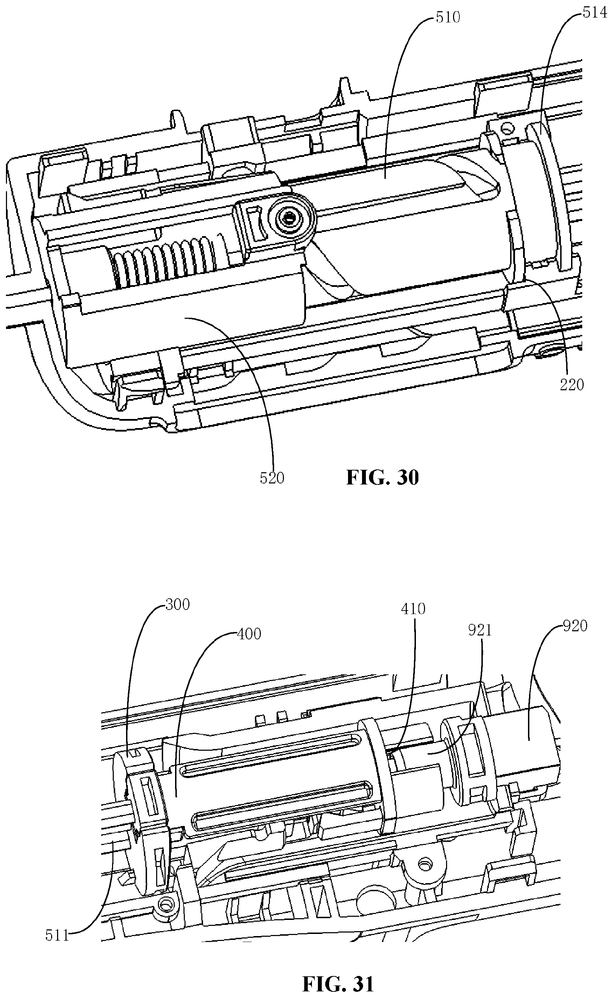

As shown in FIG. 30, it is to be understood that the rotary bracket 510 can only rotate relative to the housing 200, and cannot move along the axis of the housing 200 relative to the housing 200. In the present embodiment, the peripheral side surface of an end of the rotary bracket 510 near the insertion key 511 is provided with two flanges 514 that are spaced apart. The housing 200 is provided with an annular groove 220 corresponding to the two flanges 514. The flanges 514 are engaged in the annular groove 220. Such a configuration is for achieving the rotation of the rotary bracket 510 relative to the housing 200, and the limitation of the rotary bracket 510 relative to the housing 200 in an axial direction.

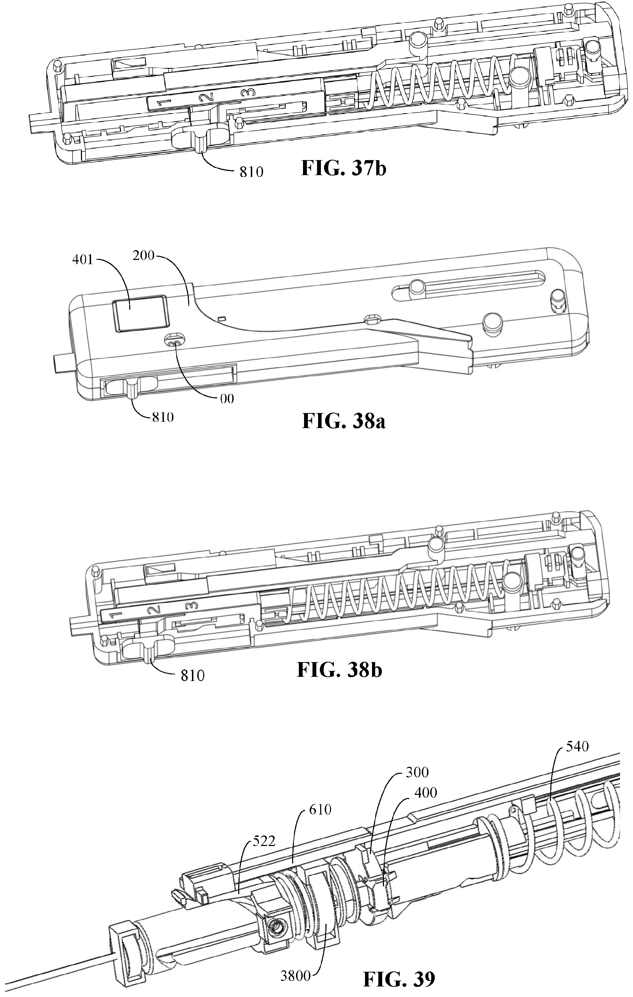

In some embodiments, the rotary bracket 510 is rotatably connected to the housing 200 via a bearing 3800 (please refer to FIG. 39).

In one embodiment, the transmission assembly includes a moving member 520. The moving member 520 is movably disposed relative to the housing 200. The moving member 520 is drivingly connected to the rotary bracket 510, and the driving assembly drives the rotary bracket 510 to rotate via the moving member 520.

Driven by the driving assembly acting on the moving member 520, the moving member 520 may drive the rotary bracket 510 to rotate. The rotary bracket 510 may rotate relative to the housing 200, so as to drive the outer needle cannula hub 300 and the inner needle cannula hub 400 to rotate relative to the housing 200.

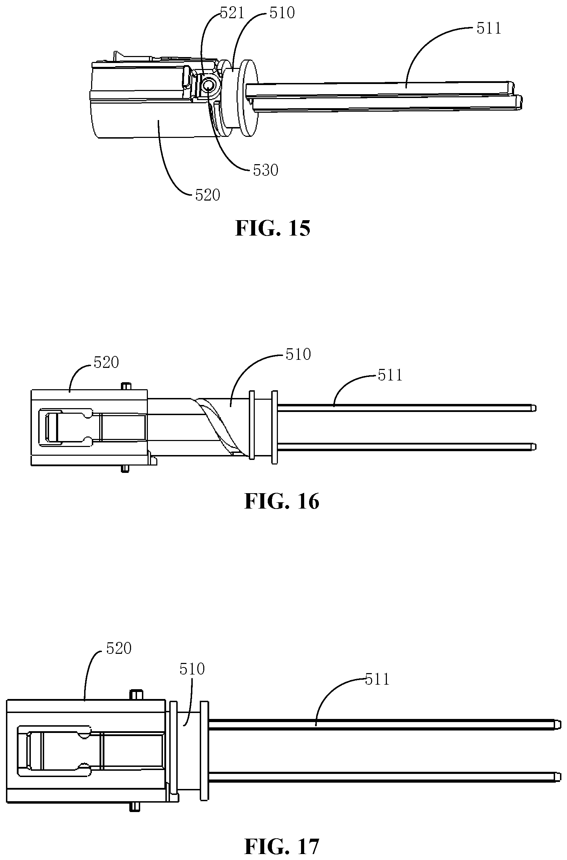

As shown in FIG. 8, FIG. 16, and FIG. 18, in one embodiment, the peripheral side surface of the rotary bracket 510 is provided with a driving sliding groove 512. The transmission assembly further includes a protruding portion 530. The protruding portion 530 is connected to the moving member 520, and a portion of the protruding portion 530 is slidably disposed in the driving sliding groove 512. When the moving member 520 moves relative to the housing 200 along a first direction, the rotary bracket 510 is driven to rotate around the axis of the housing 200 through the protruding portion 530 sliding along a trajectory of the driving sliding groove 512. The first direction refers to a direction of the moving member 520 moving along an axial direction of the housing 200 towards the distal end of the housing 200. In some embodiments, the moving member 520 is movably disposed relative to the housing 200 and is drivingly connected to the rotary bracket 510.

Configuring the moving member 520 to be movable relative to the housing 200 and drivingly connected to the rotary bracket 510 causes the protruding portion 530 to move with the moving member 520. During the process of the protruding portion 530 sliding along the trajectory of the driving sliding groove 512 on the rotary bracket 510, the rotary bracket 510 is driven to rotate relative to the housing 200 by a force of the protruding portion 530, thereby further driving the inner needle cannula hub 400 and the outer needle cannula hub 300 to rotate relative to the housing 200. Specifically, the moving member 520 is configured as a substantially cylindrical structure. The moving member 520 is at least partially sleeved on the rotary bracket 510. The moving member 520 is capable of moving along an axis of the rotary bracket 510.

As shown in FIG. 1, FIG. 3, FIG. 5 to FIG. 7, and FIG. 27, in one embodiment, the biopsy needle further includes a cocking mechanism 700. The cocking mechanism 700 includes a cocking trigger 710 rotatably connected to the housing 200 and a driving rod 720 movably disposed in the housing 200 and drivingly connected to the cocking trigger 710. The movement of the driving rod 720 along the axis of the housing 200 is driven by the rotation of the cocking trigger 710. A first stopper 721 and a second stopper 722 spaced apart from each other are configured on the driving rod 720 along an extending direction of the driving rod 720. The first stopper 721 is for connecting to the moving member 520. The second stopper 722 is for connecting to the outer needle cannula hub 300 and/or the inner needle cannula hub 400. By driving the driving rod 720 to move along the axis of the housing 200 through the rotation of the cocking trigger 710, the moving member 520 is driven to move, and the outer needle cannula hub 300 and/or the inner needle cannula hub 400 are driven to move.

Specifically, the cocking trigger 710 is rotatably connected to the housing 200 via a rotating shaft 730. The cocking trigger 710 is provided with a guiding slot. One end of the driving rod 720 is provided with a guiding protrusion 723. The guiding protrusion 723 is movably disposed in the guiding slot. When the cocking trigger 710 rotates around the rotating shaft 730, the driving rod 720 moves along the axis of the housing 200 under a pulling force of the cocking trigger 710. The first stopper 721 on the driving rod 720 applies a force to the moving member 520 to push the moving member 520 to move along the axis of the housing 200. The second stopper 722 on the driving rod 720 applies a force to the outer needle cannula hub 300 and/or the inner needle cannula hub 400 to push the outer needle cannula hub 300 and the inner needle cannula hub 400 to move simultaneously relative to the housing 200, thereby achieving the cocking of the biopsy needle.

As shown in FIG. 6, FIG. 7, FIG. 8, and FIG. 19, in one embodiment, the moving member 520 is provided with a first latch 522. After the moving member 520 moves a first preset stroke along the axial direction of the housing 200 towards the proximal end of the housing 200, the first latch 522 is latched with the housing 200. The biopsy needle further includes a first unlocking member 600. The first unlocking member 600 is movably connected to the housing 200, and the first unlocking member 600 is operably connected to the first latch 522 for operably driving the first latch 522 to disengage from the housing 200.

During the cocking process, after the first stopper 721 on the driving rod 720 pushes the moving member 520 to move the first preset stroke along the axial direction of the housing 200, the first latch 522 on the moving member 520 is latched with the housing 200.

It is to be noted that the rotary transmission mechanism 500 further includes a first driving member 540. Specifically, the first driving member 540 is a spring. The two ends of the first driving member 540 respectively abut against the moving member 520 and the rotary bracket 510. The first driving member 540 is capable of providing a driving force for the moving member 520 to move along the axial direction of the housing 200. During the process of the driving rod 720 driving the moving member 520 to move towards the proximal end of the housing 200, the first driving member 540 is compressed under the force of the moving member 520 moving along a second direction. The second direction is opposite to the first direction. After the first latch 522 is latched with the housing 200, the compressed first driving member 540 stores elastic potential energy, so as to provide kinetic energy for the moving member 520 to move relative to the housing 200 along the first direction.

In some embodiments, please refer to FIG. 39, when the rotary bracket 510 is rotatably connected to the housing 200 via the bearing 3800, one end of the first driving member 540 abuts against the moving member 520. The other end of the first driving member 540 abuts against the outer ring of a bearing 3700. This enables a rotation of the rotary bracket 510 not to affect a telescopic movement of the first driving member, or in other words, the telescopic movement of the first driving member does not affect the rotation of the rotary bracket 510.

Because the moving member 520 causes the first latch 522 to be latched with the housing 200 under the force of the driving rod 720, this ensures that the rotary transmission mechanism 500 always remains in a locked state when the first latch 522 is not subjected to an external force. In the present embodiment, by movably connecting the first unlocking member 600 to the housing 200, when a force is applied to the first unlocking member 600, the first unlocking member 600 applies a force to the first latch 522 to cause the first latch 522 to disengage from being latched with the housing 200. This thereby achieves the unlocking of the rotary transmission mechanism 500. After being unlocked, the moving member 520 in the rotary transmission mechanism 500 moves along the first direction of the housing 200 under the restoring force of the first spring, thereby driving the rotary bracket 510 to rotate relative to the housing 200 via the moving member 520.

Specifically, the first unlocking member 600 includes a connecting rod 610, a first unlocking button 620, and a second unlocking button 630. The two unlocking buttons are respectively connected to the two ends of the connecting rod 610. The first unlocking button 620 is exposed from the proximal end of the housing 200. The second unlocking button 630 is exposed from the housing 200 at a position where the first latch 522 is latched with the housing 200. The second unlocking button 630 abuts against the first latch 522. A side of the first latch 522 facing the second unlocking button 630 is configured as an inclined surface. When the second unlocking button 630 is manually pressed, the second unlocking button 630 is capable of applying a force to the first latch 522 to cause the first latch 522 to move towards the axial direction of the housing 200, thereby causing the first latch 522 to disengage from being latched with the housing 200. Alternatively, the first unlocking button 620 may also be pressed from the proximal end of the housing 200 to cause the connecting rod 610 to move along the axial direction of the housing 200 towards the distal end of the housing 200. In this case, the second unlocking button 630 can also apply a force to the first latch 522 to unlock the first latch 522. By configuring the first unlocking member 600 in a manner such that the first unlocking member 600 can be pressed from both the proximal end and the distal end of the housing 200 to unlock the rotary transmission mechanism 500, the convenience of unlocking the rotary transmission mechanism 500 is improved, so as to adapt to different operators' operating preferences.

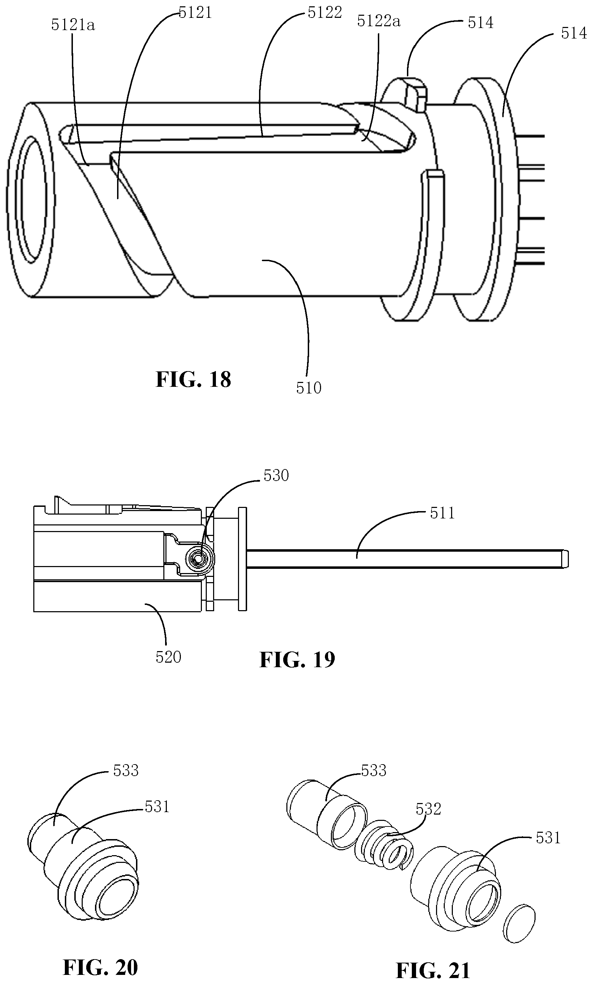

As shown in FIG. 18, in one embodiment, the driving sliding groove 512 includes the curved groove section 5122. The moving member 520 moves along the first direction, and the protruding portion 530 slides along the curved groove section 5122 to drive the rotary bracket 510 to rotate. The driving sliding groove 512 is configured to include the curved groove section 5122. When the moving member 520 moves relative to the housing 200 along the first direction, the rotary bracket 510 is driven to rotate forward relative to the housing 200 by the protruding portion 530 sliding in the curved groove section 5122. When the moving member 520 moves relative to the housing 200 along the second direction, the rotary bracket 510 is driven to rotate in reverse relative to the housing 200 by the protruding portion 530 sliding in the curved groove section 5122. In this embodiment, a trajectory of the curved groove section 5122 may be helical, or it may be other forms of curvilinear shape.

As shown in FIG. 18, in another embodiment, the driving sliding groove 512 includes a linear groove section 5121 extending along the axial direction of the rotary bracket 510, and a curved groove section 5122 extending helically around the axial direction of the rotary bracket 510. Two ends of the curved groove section 5122 are correspondingly connected to two ends of the linear groove section 5121. That is, the curved groove section 5122 and the linear groove section 5121 communicate with each other to form a closed driving sliding groove 512. The linear groove section 5121 extending along the axial direction of the rotary bracket 510 is used to guide the movement of the moving member 520 when the driving rod 720 moves relative to the housing 200 to drive the moving member 520 to move relative to the housing 200 during the cocking process. The curved groove section 5122 is used to drive the rotary bracket 510 to rotate relative to the housing 200 by the protruding portion 530 sliding along the curved groove section 5122, after the rotary transmission mechanism 500 is unlocked. It should be noted that, during the entire process, since the moving member 520 can only move along the axis of the housing 200 but cannot rotate relative to the housing 200, the moving member 520 moves toward the distal end of the housing 200 under the elastic restoring force of the first driving member 540, that is, moves along the first direction, and the protruding portion 530 drives the rotary bracket 510 to rotate through the curved groove section 5122 as the moving member 520 moves along the axis of the housing 200.

In one embodiment, each of the linear groove section 5121 and the curved groove section 5122 includes a head end and a tail end. The head end is an end facing the proximal end of the housing 200, and the tail end is an end facing the distal end of the housing 200. Both the head end of the linear groove section 5121 and the tail end of the curved groove section 5122 are provided with a stop structure, or both the tail end of the linear groove section 5121 and the head end of the curved groove section 5122 are provided with the stop structure. The stop structure is configured to prevent the protruding portion 530 from retreating after the protruding portion 530 passes through the stop structure from one direction.

By configuring the stop structure, the protruding portion 530 is blocked to prevent the protruding portion 530 from retreating when the protruding portion 530 moves along one direction in the driving sliding groove 512. This ensures that the protruding portion 530 can only move along one direction in the driving sliding groove 512, thereby guaranteeing that the rotary bracket 510 can only rotate relative to the housing 200 along one direction, and ensuring reliability during tissue cutting.

It should be understood that the specific structure of the stop structure is not limited. Specifically, the stop structure may adopt the following structural forms.

As shown in FIG. 8 to FIG. 18, in one embodiment, the stop structure at the head end of the linear groove section 5121 is a stepped structure formed by a groove bottom of the head end of the linear groove section 5121 higher than a groove bottom of the head end of the curved groove section 5122, so as to prevent the protruding portion 530 from retreating after the protruding portion 530 moves from the head end of the linear groove section 5121 into the curved groove section 5122. The stop structure at the tail end of the curved groove section 5122 is a stepped structure formed by a groove bottom of the tail end of the curved groove section 5122 higher than the tail end of the linear groove section 5121, so as to prevent the protruding portion 530 from retreating after the protruding portion 530 enters the tail end of the linear groove section 5121 from the tail end of the curved groove section 5122. The groove bottom of the curved groove section 5122 and the groove bottom of the linear groove section 5121 are smooth surfaces.

When the protruding portion 530 slides along the extending direction of the linear groove section 5121 into the curved groove section 5122, since the head end of the linear groove section 5121 is provided with a first limiting step 5121a having a groove bottom higher than the groove bottom of the head end of the curved groove section 5122, the protruding portion 530 can be prevented from retreating from the head end of the curved groove section 5122 to the head end of the linear groove section 5121 by the stepped structure. when the protruding portion 530 slides along an extending direction of the curved groove section 5122 into the linear groove section 5121, since the tail end of the curved groove section 5122 is provided with a second limiting step 5122a having a groove bottom higher than the groove bottom of the tail end of the linear groove section 5121, the protruding portion 530 can be prevented from retreating from the tail end of the linear groove section 5121 to the tail end of the curved groove section 5122 by the stepped structure. It should be noted that, in this embodiment, in addition to the stepped structure, the groove bottoms of other parts of the linear groove section 5121 and the curved groove section 5122 are configured as smooth surfaces.

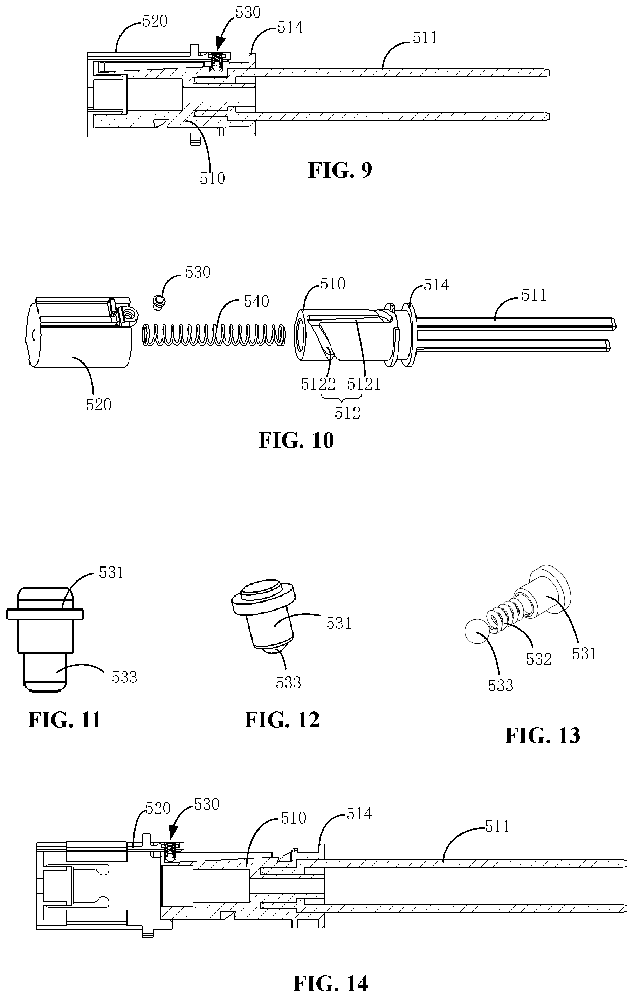

As shown in FIG. 11, FIG. 12, FIG. 13, FIG. 20, and FIG. 21, the protruding portion 530 includes a support base 531, a second driving member 532, and a movable member 533. The support base 531 is configured with an accommodating chamber having an opening at one end, and the support base 531 is fixedly connected to the moving member 520. The second driving member 532 is disposed in the accommodating chamber, and one end of the second driving member 532 abuts against the support base 531. The movable member 533 is movably disposed in the accommodating chamber and abuts against another end of the second driving member 532, wherein a portion of the movable member 533 protrudes from an end face of the support base 531.

The support base 531 is used to connect to the moving member 520 and also to support the second driving member 532 and the movable member 533. In this embodiment, the movable member 533 is a ball, and the second driving member 532 is a spring. One end of the accommodating chamber of the support base 531 is provided with a through hole having a diameter smaller than the diameter of the ball. The ball is disposed in the accommodating chamber and partially protrudes from the end face of the support base 531 through the through hole. One end of the second driving member 532 abuts against the ball, and another end abuts against a cover plate of the support base 531. When the protruding portion 530 slides along the driving sliding groove 512 under the drive of the moving member 520, the ball rolls in the driving sliding groove 512.

Stepped structures are provided at both the head end of the linear groove section 5121 and the tail end of the curved groove section 5122, so that when the protruding portion 530 slides along the driving sliding groove 512 and passes through the stepped structures, the protruding portion 530 can smoothly pass through groove bottoms of different depths, and is reliably stopped at the stepped structures without retreating. In this embodiment, by providing the second driving member 532, when the protruding portion 530 passes through the stepped structure, the ball smoothly passes over the stepped structure through the expansion and contraction of the second driving member 532. At the same time, the second driving member 532 always has a tendency to move the ball outward along an axis of the support base 531, so that when the ball passes over the stepped structure, it is not easy to retreat under a driving force of the second driving member 532 and a blocking action of the stepped structure. Certainly, in some embodiments, to enable the protruding portion 530 to more easily pass over the stepped structure while also better blocking the protruding portion 530, the stepped structure is configured as a ramp form.

In another embodiment, the height of the groove bottom of the linear groove section 5121 may also be configured such that it gradually increases from the tail end of the linear groove section 5121 toward the head end of the linear groove section 5121, and the groove bottom of the head end of the curved groove section 5122 is lower than the groove bottom of the head end of the linear groove section 5121. Furthermore, the height of the groove bottom of the curved groove section 5122 may be configured such that it gradually increases from the head end of the curved groove section 5122 toward the tail end of the curved groove section 5122, and the groove bottom of the tail end of the curved groove section 5122 is higher than the groove bottom of the tail end of the linear groove section 5121.

Such a configuration causes a stop step to be formed by a height difference of the groove bottoms at the head end of the linear groove section 5121 and the tail end of the curved groove section 5122, to block the protruding portion 530 and thus prevent the protruding portion 530 from retreating.

It can be understood that, in this embodiment, since both the linear groove section 5121 and the curved groove section 5122 are configured as ramp forms, their groove bottoms are smoothly arranged, and the friction force between the groove bottoms and the movable member 533 is relatively small; thus, the movable member 533 is configured as a cylindrical structure. Effective stopping of the protruding portion 530 when it enters the curved groove section 5122 from the linear groove section 5121 of the rotary bracket 510 mainly relies on a step formed by a height difference between the groove bottom of the head end of the linear groove section 5121 and the groove bottom of the head end of the curved groove section 5122. The cylindrical movable member 533 achieves expansion and contraction with the aid of the compression and elastic force of the second driving member 532. The large expansion and contraction amount can better meet the movement and stopping requirements of the protruding portion 530 within the linear groove section 5121 and the curved groove section 5122.

Furthermore, an accommodating groove 521 is provided on a peripheral side surface of the end of the moving member 520 near the rotary bracket 510, and a mounting hole for the protruding portion 530 to pass through is provided in a groove bottom of the accommodating groove 521. One end of the protruding portion 530 is disposed in the accommodating groove 521, and another end protrudes from the mounting hole and extends into the driving sliding groove 512 on the rotary bracket 510. The end of the portion of the protruding portion 530 located in the accommodating groove 521 is at most flush with the peripheral side surface of the moving member 520. Such a configuration, while fixing the protruding portion 530, ensures that the protruding portion 530 does not protrude beyond the peripheral side surface of the moving member 520. This allows the protruding portion 530 to have a certain expansion and contraction space during the movement of the moving member 520 relative to the housing 200, even when the protruding portion 530 expands and contracts with changes in the depth of the groove bottom, thus preventing interference with the housing 200, which could lead to excessive friction and difficult movement.

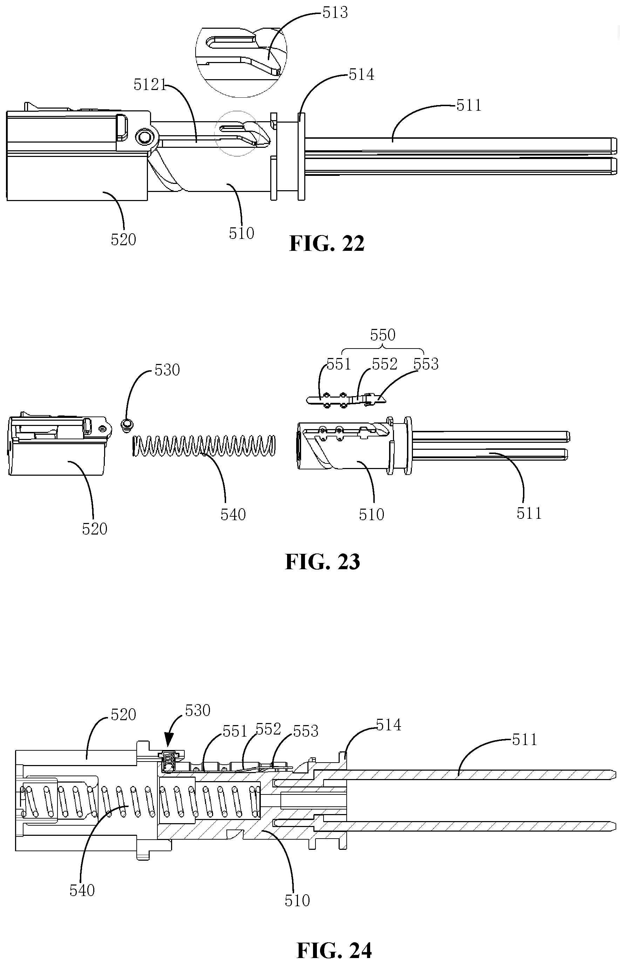

As shown in FIG. 22 to FIG. 26, in one embodiment, the stop structure at the head end of the linear groove section 5121 is a biasing member. The biasing member is at least partially disposed in the linear groove section 5121. When the protruding portion 530 moves past the biasing member along the second direction, the biasing member is biased to avoid the protruding portion 530. When the protruding portion 530 moves into the curved groove section 5122, the biasing member resets to block the protruding portion 530 from retreating. The first direction is opposite to the second direction. By providing the biasing member at the head end of the linear groove section 5121, the deformation of the biasing member enables the protruding portion 530 to smoothly move from the head end of the linear groove section 5121 into the curved groove section 5122. The rebound of the biasing member blocks the protruding portion 530, thereby preventing the protruding portion 530 from retreating.

As shown in FIG. 22, specifically, in some embodiments, the biasing member is a latch force arm 513 provided at the head end of the linear groove section 5121. The latch force arm 513 is disposed on a groove side wall of the linear groove section 5121, and an end of the latch force arm 513 is biased inwardly into the linear groove section 5121. A recess is provided on the peripheral side surface of the rotary bracket 510 at a position adjacent to the latch force arm 513, which provides the latch force arm 513 with certain elastic deformation capability. When the protruding portion 530 passes the latch force arm 513, the latch force arm 513 is able to move toward the recess under the action of the protruding portion 530, so that the portion of the linear groove section 5121 provided with the latch force arm 513 is opened, so as to allow the protruding portion 530 to pass through and enter the curved groove section 5122. When the protruding portion 530 enters the curved groove section 5122, the latch force arm 513 rebounds and closes the head end of the linear groove section 5121. At this time, the latch force arm 513 blocks the protruding portion 530 to prevent the protruding portion 530 from retreating. This form of the driving sliding groove 512, which stops by means of the latch-type latch force arm 513 instead of the bottom interference described above, allows the linear groove section 5121 in the rotary bracket 510 to be configured with a planar groove bottom.

In this structural form of the stop structure, the specific structure of the protruding portion 530 is not limited, and it does not necessarily need to have expansion and contraction properties or a large expansion and contraction amount. The stop structure of this configuration mainly relies on the deformation of the door-opening type latch force arm 513 to yield to the protruding portion 530. The door-closing type latch force arm 513 rebounds to laterally block the straight return path of the protruding portion 530. A large expansion and contraction amount of the protruding portion 530 is not required. Therefore, in this embodiment, the protruding portion 530 adopts a ball micro-expansion/contraction scheme to reduce kinetic friction. Alternatively, in other embodiments, the protruding portion 530 may not have an expansion and contraction function. The protruding portion 530 only needs to be a protrusion that does not abut the groove bottom, maintains a certain distance from the groove bottom, and does not generate frictional resistance with the groove bottom.

As shown in FIG. 23 to FIG. 26, in other embodiments, the biasing member is a first elastic sheet 550. The first elastic sheet 550 is disposed in the linear groove section 5121 and includes a connecting portion 551, a transition portion 552, and a stopping portion 553 sequentially arranged. The connecting portion 551 is fixedly connected to the rotary bracket 510. The transition portion 552 is obliquely disposed, and an end of the transition portion 552 close to the stopping portion 553 is away from the groove bottom of the linear groove section 5121, so that the stopping portion 553 protrudes upwardly away from the groove bottom of the linear groove section 5121. The transition portion 552 and the stopping portion 553 are movable toward the groove bottom of the linear groove section 5121 under the pressure of a guiding assembly, to enable the guiding assembly to move toward the curved groove section 5122.

Specifically, the first elastic sheet 550 may be fixed on the groove bottom of the linear groove section 5121 by means of hot melt bonding or spot gluing. By configuring the first elastic sheet 550 in the form of the connecting portion 551, the transition portion 552, and the stopping portion 553, when the protruding portion 530 slides in the linear groove section 5121, the first elastic sheet 550 is smoothly and progressively compressed, thereby allowing the protruding portion 530 to smoothly enter the curved groove section 5122 from the linear groove section 5121. When the protruding portion 530 falls into the curved groove section 5122, the stopping portion 553 of the first elastic sheet 550 rebounds, which can effectively block the side surface of the protruding portion 530, thereby preventing the protruding portion 530 from retreating along the linear groove section 5121. In this embodiment, the protruding portion 530 may not have expansion and contraction properties.

As shown in FIG. 23 to FIG. 26, furthermore, a barb structure is provided on the connecting portion 551 of the first elastic sheet 550, which can be supported on the bottom of the linear groove section 5121 to prevent the first elastic sheet 550 from collapsing. At the same time, the barb structure is disposed in the linear groove section 5121, which can prevent the first elastic sheet 550 from inverting, ensure the effective height of the first elastic sheet 550, and thereby ensure the reliability of the stopping of the first elastic sheet 550.

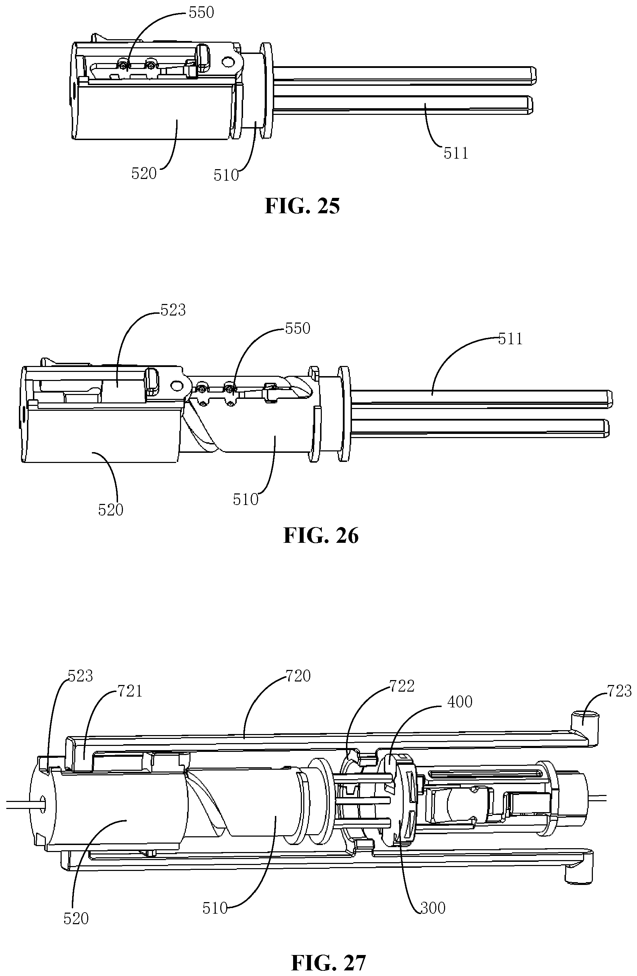

As shown in FIG. 26 and FIG. 27, in one embodiment, the moving member 520 is provided with a stroke avoidance groove 523, and the first stopper 721 is disposed in the stroke avoidance groove 523, such that the first stopper 721 abuts against the moving member 520 after the driving rod 720 moves a second preset stroke driven by the cocking trigger 710, thereby driving the moving member 520 to move.

During the cocking process, the second stopper 722 of the driving rod 720 first pushes the inner needle cannula hub 400 and the outer needle cannula hub 300 to move along the axis of the housing 200 toward the proximal end of the housing 200 for a second preset stroke, which corresponds to the length of the stroke avoidance groove 523. After that, the first stopper 721 of the driving rod 720 abuts against the moving member 520. When the cocking trigger pulls the driving rod 720 to continue moving along the axis of the housing 200 toward the proximal end of the housing 200, the driving rod 720 simultaneously pushes the moving member 520 and the inner/outer needle cannula hubs to move along the axis of the housing 200 toward the proximal end of the housing 200. After the driving rod 720 pushes the moving member 520 and the inner/outer needle cannula hubs to move the first preset stroke, the first latch 522 on the moving member 520 is latched with the housing 200. The second latch 310 on the outer needle cannula hub 300 is also latched with the housing 200, and the cocking is completed at this time.

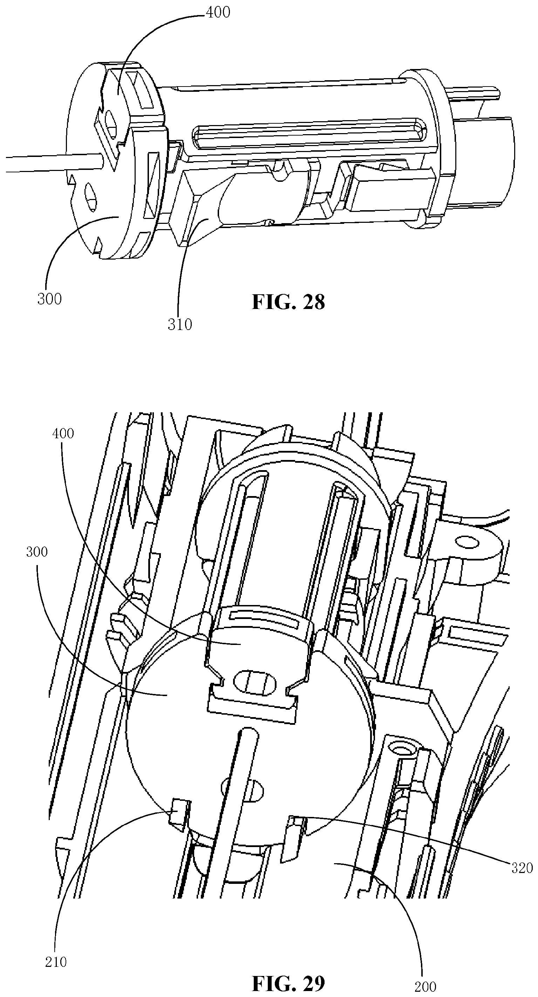

As shown in FIG. 28, furthermore, the inner needle cannula hub 400 and the outer needle cannula hub 300 are movably connected relative to each other. A second latch 310 is provided on the outer needle cannula hub 300 and/or the inner needle cannula hub 400. After the outer needle cannula hub 300 and/or the inner needle cannula hub 400 moves a third preset stroke toward the proximal end of the housing 200 from a firing completed position, the second latch 310 is latched with the housing 200, and cocking is completed. It can be understood that the sum of the first preset stroke and the second preset stroke is at least equal to the third preset stroke.

As shown in FIG. 29, in one embodiment, a locking slot 320 is disposed on a peripheral side surface of the outer needle cannula hub 300 and/or a peripheral side surface of the inner needle cannula hub 400. A locking protrusion 210 is disposed on an inner wall of the housing 200. When the second latch 310 is latched with the housing 200, the locking protrusion 210 is engaged in the locking slot 320. When the second latch 310 is latched with the housing 200, the locking protrusion 210 is engaged in the locking slot 320 on the housing 200, thereby locking the rotation of the rotary transmission mechanism 500. This ensures that after the biopsy needle is cocked and before firing, the rotary transmission mechanism 500 cannot be driven, thus ensuring that the inner needle cannula hub 400 and the outer needle cannula hub 300 do not rotate.

Specifically, the locking slot 320 extends along the axial direction of the housing 200, and the locking protrusion 210 also extends along the axial direction of the housing 200. Thus, after the biopsy needle is fired, during the movement of the inner needle cannula hub 400 and the outer needle cannula hub 300 along the axial direction of the housing 200 toward the distal end of the housing 200, the locking protrusion 210 can disengage from the locking slot 320, thereby enabling the rotary bracket 510 to rotate relative to the housing 200 after the rotary transmission mechanism 500 is unlocked.

As shown in FIG. 27, in one embodiment, a count of driving rods 720 is two. The two driving rods 720 are disposed opposite to each other along a radial direction of the moving member 520, and the second stoppers 722 on the two driving rods 720 are interlocked with each other. By providing two opposing driving rods 720 in the radial direction of the moving member 520, a pushing force is applied to the moving member 520 by the first stoppers 721 on the two driving rods 720, and a pushing force is applied to the outer needle cannula hub 300 by the two second stoppers 722. This prevents the moving member 520, the inner needle cannula hub 400, and the outer needle cannula hub 300 from shaking during the cocking process. Specifically, the second stopper 722 is configured with an arc shape. The opposing ends of the two second stoppers 722 are provided with interlocked protrusions and recesses. The two second stoppers 722 are interlocked by means of male-female engagement. The interlocking of the two second stoppers 722 improves their stability, thereby improving the stability of the movement of the driving rod 720.

As shown in FIG. 31, in one embodiment, the biopsy needle further includes the core needle 110 and a core needle seat 920. The core needle seat 920 is fixedly connected to the proximal end of the housing 200. The core needle 110 is fixedly connected to the core needle seat 920. An end of the core needle seat 920 facing the distal end of the housing 200 is provided with a first guiding portion. An end of the inner needle cannula hub 400 or the outer needle cannula hub 300 facing the proximal end of the housing 200 is provided with a second guiding portion. When the second latch 310 is latched with the housing 200, the first guiding portion and the second guiding portion are engaged with each other.

Engaged connection of the first guiding portion and the second guiding portion makes the inner needle cannula hub 400 and the outer needle cannula hub 300 more stable when cocking is completed. When the inner needle cannula hub 400 and the outer needle cannula hub 300 are always connected to the insertion key during a cocking and firing process, and at the same time the first guiding portion and the second guiding portion are engaged, a requirement for structural strength of the insertion key 511 on the rotary bracket 510 is reduced, and a certain guiding effect can be achieved. This thereby improves the stability of the operation of the rotary transmission mechanism 500. Specifically, a guiding channel 410 is provided at the end of the outer needle cannula hub 300 or the inner needle cannula hub 400 facing the proximal end. The guiding channel 410 is the second guiding portion. A guiding protrusion 921 is provided at the end of the core needle seat 920 facing the distal end of the housing 200. The guiding protrusion 921 is the first guiding portion. During the movement of the outer needle cannula hub 300 and the inner needle cannula hub 400 toward the proximal end of the housing 200, the guiding protrusion 921 may be inserted into the guiding channel 410. At this time, the insertion key 511 is located in an insertion slot formed by the inner needle cannula hub 400 and/or the outer needle cannula hub 300. Through the guiding of the insertion key 511 and the engaged guiding of the first guiding portion and the second guiding portion, the skewed movement of the inner/outer needle cannula hubs can be prevented.

In one embodiment, the biopsy needle further includes a shifting mechanism 800, and the shifting mechanism 800 includes a first shifting member 820, a second shifting member, and a gear shift button 810. The first shifting member 820 is slidably connected to the insertion key 511 for limiting the moving position of the inner needle cannula hub 400 and the outer needle cannula hub 300. The second shifting member is movably disposed in the housing 200 and rotatably connected to the first shifting member 820, and movement of the second shifting member is capable of controlling the position of the first shifting member 820 relative to the insertion key 511. The gear shift button 810 is disposed on the housing 200 and connected to the second shifting member, and the gear shift button 810 is operable to move relative to the housing 200 to control the position of the first shifting member through the second shifting member. By controlling the position of the first shifting member relative to the insertion key through the second shifting member, the stroke of the outer needle cannula hub 300 and the inner needle cannula hub 400 after firing is achieved, thereby controlling the sampling distance.

Specifically, the first shifting member is provided with two stop surfaces, namely a protruding platform and a large end face. The protruding platform is for stopping the inner needle cannula hub 400, and the large end face is for stopping the outer needle cannula hub 300. Additionally, a small spring is disposed between the inner and outer needle cannula hubs to maintain a displacement distance between the inner and outer needle cannula hubs. During the movement of the inner and outer needle cannula hubs in the sampling firing process, the third driving member 910 pushes the inner needle cannula hub 400. The inner needle cannula hub 400 then pushes the outer needle cannula hub 300 to move from the proximal end to the distal end of the housing 200. Because the elastic force of the third driving member 910 is far greater than the elastic force of the small spring between the inner and outer needle cannula hubs, the outer needle cannula hub 300 and the inner needle cannula hub 400 remain relatively static during the movement of the inner and outer needle cannula hubs being fired. This maintains a state where the second elastic sheet 131 of the outer needle cannula 130 is not inserted into the slot of the inner needle cannula 120. When the inner needle cannula hub 400 is stopped by the protruding platform, the outer needle cannula hub 300 continues to move forward under the inertia of the small spring and the outer needle cannula hub 300 until the outer needle cannula hub 300 is stopped by the large end face. During this process, the inner and outer needle cannula hubs move relatively, thereby causing the second elastic sheet 131 of the outer needle cannula 130 to be inserted into the slot of the inner needle cannula 120.

It should be noted that, in some embodiments, a latch is disposed at the distal end of the inner needle cannula hub 400. The latch abuts against the distal end of the outer needle cannula hub 300, so that when the third driving member 910 pushes the outer needle cannula hub 300 to move, the inner needle cannula hub 400 is capable of moving together with the outer needle cannula hub 300 simultaneously. During this process, the cutting edge at the distal end of the inner needle cannula 120 penetrates into tissue, so that the target tissue enters the inner needle cannula 120, and the outer needle cannula 130 moves simultaneously with the inner needle cannula. When the inner needle cannula hub 400 moves to abut against the protruding platform and is stopped, the latch engagement on the inner needle cannula hub 400 can be simultaneously unlocked. The outer needle cannula hub 300 disengages from the latch engagement on the inner needle cannula hub 400 and continues to move a certain distance towards the distal end of the housing under the elastic force of the third driving member 910. At this time, the second elastic sheet 131 on the outer needle cannula 130 enters the inner needle cannula 120 from a slot 3521 on the inner needle cannula 120, passes through the sample tissue in the inner needle cannula, and achieves the purpose of radially cutting the sample tissue. This process is the firing process of the biopsy needle.

It can be understood that, in a technical solution where the elastic blade is disposed on the inner needle cannula 120 and two spaced-apart slots are disposed on the outer needle cannula 130, a latch is disposed at the distal end of the outer needle cannula hub 300. The latch abuts against the distal end of the inner needle cannula hub 400, and the third driving member 910 abuts against the proximal end of the inner needle cannula hub 400. When the third driving member 910 pushes the inner needle cannula hub 400 to move, the outer needle cannula hub 300 is capable of moving simultaneously with the inner needle cannula hub 400. During this process, the cutting edge at the distal end of the inner needle cannula 120 penetrates into tissue, so that the target tissue enters the inner needle cannula 120, and the outer needle cannula 130 moves simultaneously with the inner needle cannula 120. When the outer needle cannula hub 300 moves to abut against the protruding platform and is stopped, the latch on the outer needle cannula hub 300 is unlocked. The inner needle cannula hub 400 disengages from the latch engagement on the outer needle cannula hub 300 and continues to move a certain distance towards the distal end of the housing under the elastic force of the third driving member 910. During this process, the inner needle cannula 120 moves relative to the outer needle cannula 130 driven by the inner needle cannula hub 400. At this time, the elastic blade on the inner needle cannula 120 enters the outer needle cannula 130 from the slot near the distal end on the outer needle cannula 130, passes through the sample tissue between the inner needle cannula 120 and the outer needle cannula 130, and achieves the purpose of radially cutting the sample tissue.

In some other embodiments, the small spring may not be disposed between the inner and outer needle cannula hubs, and the outer needle cannula hub 300 moves relative to the inner needle cannula hub 400 completely by the inertia of the outer needle cannula hub 300 during the firing process.

It should be noted that the biopsy needle further includes a third driving member 910. The third driving member 910 is disposed in the housing 200 and sleeved on the core needle 110. One end of the third driving member 910 abuts against the housing 200, and another end abuts against the outer needle cannula hub 300 and/or the inner needle cannula hub 400. When the second latch 310 is disengaged from the housing 200, the inner needle cannula hub 400 and the outer needle cannula hub 300 move towards the distal end of the housing 200 under the elastic force of the third driving member 910. In some embodiments, the third driving member 910 may be a firing spring.

The working principle of the biopsy needle provided by the above-described embodiments is as follows (which can be understood with reference to FIG. 1 to FIG. 31).

Before inserting the biopsy needle into the human body, the biopsy needle needs to be cocked. Before the biopsy needle is cocked, the second elastic sheet 131 of the outer needle cannula 130 remains inserted into the inner needle cannula 120, and the third driving member 910 is in a relaxed state. By pulling the cocking trigger 710, the cocking trigger 710 rotates relative to the housing 200. During rotation of the cocking trigger 710, the cocking trigger 710 pulls the driving rod 720 to move along the axis of the housing 200 towards the proximal end of the housing 200. In an initial stage, after the second stopper 722 of the driving rod 720 pushes the inner needle cannula hub 400 and the outer needle cannula hub 300 to move a second preset stroke along the axis of the housing 200, the first stopper 721 of the driving rod 720 abuts against the moving member 520. After the driving rod 720 continues to move the first preset stroke towards the proximal end of the housing 200, the first latch 522 on the moving member 520 is latched with the housing 200, and the second latch 310 on the outer needle cannula hub 300 is latched with the housing 200. During the cocking process, the first driving member 540 between the moving member 520 and the rotary bracket 510 is compressed and is in a ready state. Similarly, the third driving member 910 disposed between the outer needle cannula hub 300 and the housing 200 is compressed and is in a ready state. Thus, the cocking of the biopsy needle is completed.

After cocking, the core needle 110 is in a protruding state, and the second elastic sheet 131 of the outer needle cannula 130 disengages from the curved groove of the inner needle cannula 120. Next, the gear shifting operation can be performed. The gear shift button may be toggled to adjust a desired gear. Then, guided by imaging equipment, the biopsy needle is inserted into the human body and moved near the target tissue. After toggling the second unlocking member, a firing and sampling action is performed. It should be noted that, in the technical solution of the present disclosure, the second unlocking member, similar to the first unlocking member, may be unlocked from both the distal end and the proximal end of the housing. This includes a front firing button and a tail firing button.

Specifically, a doctor can press the tail firing button at the tail end. The protruding platform structure in the button may push open the second latch 310 on the outer needle cannula hub, causing the second latch 310 to disengage from the housing 200. At this time, the compressed third driving member 910 may release energy, converting the potential energy accumulated from previous compression into kinetic energy, and pushing the outer needle cannula hub 300 and the inner needle cannula hub 400 to move along the axis of the housing 200 towards the distal end of the housing 200. Because the inner and outer needle cannula hubs are capable of moving relatively to each other, when the inner needle cannula hub 400 abuts against the first shifting member, the protruding platform on the first shifting member may first stop the inner needle cannula hub 400. Then, the large end face of the first shifting member may stop the outer needle cannula hub, so as to ensure that the second elastic sheet 131 on the outer needle cannula 130 extends into the curved groove of the inner needle cannula 120. Specifically, the inner and outer needle cannula hubs may simultaneously reach the first shifting member at the same speed. The movement stops when the inner needle cannula hub 400 collides with the protruding platform of the first shifting member, thereby completing the preliminary cutting of the target tissue by the inner needle cannula 120 driven by the inner needle cannula hub 400. At this time, the outer needle cannula hub 300 continues to move forward for a certain distance under the action of inertia until it hits the stop surface of the first shifting member and stops, thereby completing the entry of the second elastic sheet 131 of the outer needle cannula 130, driven by the outer needle cannula hub 300, into the slot of the inner needle cannula 120 to cut off the target tissue within the inner needle cannula 120.

Of course, during the firing process, the doctor can also operate the front firing button, which may be linked with the tail firing button to fire the inner and outer needle cannula hubs. The specific firing process is the same as the above-described movement process.

After the biopsy needle completes firing, the first unlocking member 600 is pressed to unlock the rotary transmission mechanism 500, causing the first latch 522 to disengage from the housing 200. The moving member 520 moves towards the distal end of the housing 200 under the elastic force of the first driving member 540. The protruding portion 530 moves along the curved groove section 5122 of the driving sliding groove 512 to push the rotary bracket 510 to rotate relative to the housing 200. Since the insertion key is always connected to the inner and outer needle cannula hubs, the inner and outer needle cannula hubs rotate relative to the housing 200, driven by the rotary bracket 510, thereby driving the inner needle cannula 120 and the outer needle cannula 130 to rotate relative to the core needle 110. During the rotation of the inner needle cannula hub 400, the second elastic sheet 131 on the outer needle cannula 130, extending into the inner needle cannula 120, may also rotate together to circumferentially cut the sample tissue within the inner needle cannula 120 by the second elastic sheet 131, thereby achieving the purpose of separating the sample tissue from surrounding tissue.

Regarding biopsy needle sampling, the process is to first temporarily store the target tissue in a chamber formed by the inner needle cannula and the core needle of the biopsy needle, then insert the cutting blade on the outer needle cannula into the inner needle cannula and supplementarily cut the temporarily stored target tissue, thereby obtaining the target tissue sample completely separated from human tissue, and then withdraw the needle body.

During the sampling process, when the cutting blade is inserted into the inner needle cannula for supplementary cutting, the target tissue cannot be completely cut off. This causes pulling damage to the tissue during the removal of the target tissue sample due to tissue entanglement, and even leads to a problem where the target tissue sample detaches from the needle tube under pulling, resulting in sampling failure.

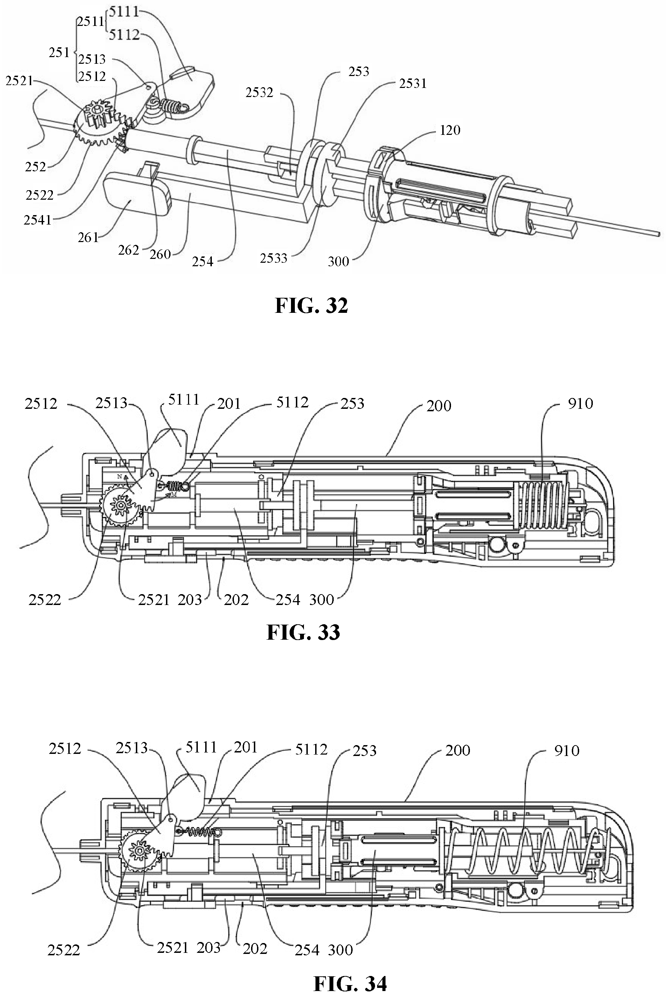

Please refer to FIG. 32, FIG. 32 is a partial structural diagram of the biopsy needle according to some embodiments of the present disclosure.

In another embodiment of the present disclosure, a biopsy needle is provided, including the housing 200, the inner needle cannula 120, the outer needle cannula 130, and the rotary transmission mechanism.

The rotary transmission mechanism is disposed in the housing 200, and includes a driving assembly 251 and a transmission assembly drivingly connected to the driving assembly 251.

Please refer to FIG. 33 and FIG. 34 together, FIG. 33 is a schematic diagram illustrating an internal structure of the biopsy needle (without completing sampling firing) according to some embodiments of the present disclosure, and FIG. 34 is a schematic diagram illustrating an internal structure of the biopsy needle (after completion of sampling firing) according to some embodiments of the present disclosure.

After the inner needle cannula hub 400 and the outer needle cannula hub 300 are fired to complete sampling, for example, after the inner needle cannula hub 400 and the outer needle cannula hub 300 respectively move a preset stroke along the axial direction of the inner needle cannula 120 to complete sampling firing, the driving assembly is operated, thereby operably driving the rotary transmission mechanism a plurality of times, enabling the plurality of synchronous rotations of the inner needle cannula hub 400 and the outer needle cannula hub 300.

After the inner needle cannula hub 400 and the outer needle cannula hub 300 are fired to complete sampling, both the inner needle cannula hub 400 and the outer needle cannula hub 300 are connected to the transmission assembly, and the second elastic sheet 131 of the outer needle cannula 130 is inserted into the slot of the inner needle cannula 120. By operably driving the rotary transmission mechanism a plurality of times, enabling the plurality of synchronous rotations of the inner needle cannula hub 400 and the outer needle cannula hub 300, so as to allow the second elastic sheet 131 to rotate around the axis of the inner needle cannula 120 to perform secondary cutting on the target tissue, thereby enabling a more thorough cutting of the target tissue to obtain a target tissue sample completely separated from human tissue.

It is understandable that, considering that if both the inner needle cannula hub 400 and the outer needle cannula hub 300 are connected to the transmission assembly again during the firing process, it is necessary to align and connect the moving inner needle cannula hub 400 and the moving outer needle cannula hub 300 to the transmission assembly, which is relatively difficult to operate and has higher design requirements. Therefore, in order to lower design precision requirements, before the inner needle cannula hub 400 and the outer needle cannula hub 300 are fired to complete sampling, both the inner needle cannula hub 400 and the outer needle cannula hub 300 may also be connected to the transmission assembly. That is, both before and after the inner needle cannula hub 400 and the outer needle cannula hub 300 are fired to complete sampling, the inner needle cannula hub 400 and the outer needle cannula hub 300 are always connected to the transmission assembly.

During the firing for sampling process of the biopsy needle, firstly, the inner needle cannula hub 400 and the outer needle cannula hub 300 synchronously move a first stroke along the axial direction of the inner needle cannula 120. During this process, the inner needle cannula 120 performs a first step of cutting on the target tissue, causing the target tissue to enter the inner needle cannula 120. Then, the inner needle cannula 120 stops moving, and the outer needle cannula 130 continues to move a second stroke along the axial direction of the inner needle cannula 120. During this process, the second elastic sheet 131 extends into the slot to perform cutting on the target tissue within the inner needle cannula 120. In the present disclosure, the sampling firing is preliminarily completed at this point.

For example, after driving the rotary transmission mechanism for the first time, enabling a first synchronous rotation of the inner needle cannula hub 400 and the outer needle cannula hub 300, the rotary transmission mechanism may be driven for a second time, enabling a second synchronous rotation of the inner needle cannula hub 400 and the outer needle cannula hub 300. After driving the rotary transmission mechanism for the K-th time, enabling a K-th synchronous rotation of the inner needle cannula hub 400 and the outer needle cannula hub 300, the rotary transmission mechanism may be driven for a (K+1)-th time, enabling a (K+1)-th synchronous rotation of the inner needle cannula hub 400 and the outer needle cannula hub 300, wherein K is a positive integer greater than or equal to 1.

The biopsy needle provided by the embodiments of the present disclosure includes the driving assembly 251 and the transmission assembly drivingly connected to the driving assembly 251. After the inner needle cannula hub 400 and the outer needle cannula hub 300 are fired to complete sampling, both the inner needle cannula hub 400 and the outer needle cannula hub 300 are connected to the transmission assembly. By operably driving the rotary transmission mechanism a plurality of times, enabling the plurality of synchronous rotations of the inner needle cannula hub 400 and the outer needle cannula hub 300, the biopsy needle can be rotated more conveniently by driving the rotary transmission mechanism to perform a more thorough cutting on the target tissue. At this point in the present disclosure, the sampling firing is completely finished to obtain a target tissue sample completely separated from human tissue. This can ameliorate pulling damage caused to the tissue when the target tissue sample is taken out due to tissue entanglement during sampling, and can even prevent problems such as the target tissue sample potentially detaching from the needle tube under pulling, leading to sampling failure.

The biopsy needle provided by the embodiments of the present disclosure integrates a flexible and convenient rotary transmission mechanism, which solves the problem that a conventional tubular needle body does not completely cut samples during puncture. It achieves more reliable sampling, enhances the cutting effect between the second elastic sheet 131 of the outer needle cannula 130 and the tissue to be cut, is conducive to successful sampling, and can repeatedly achieve a plurality of rotations as needed.

In one embodiment, the driving assembly 251 is movably or rotatably disposed on the housing 200. The transmission assembly includes the gear set 252. The driving assembly 251 is drivingly connected to the rotary bracket 510 through the gear set 252 to drive the rotary bracket 510 to rotate.

Under the action of the driving assembly 251 driving the gear set 252, the gear set 252 may drive the rotary bracket 510 to rotate. The rotary bracket 510 may rotate relative to the housing 200, so as to drive the outer needle cannula hub 300 and the inner needle cannula hub 400 to rotate relative to the housing 200.

Optionally, the transmission assembly includes the gear set 252. The gear set 252 is drivingly connected to the driving assembly 251. The rotary bracket includes a transition member 253 and a transmission member 254. The transition member 253 is rotatable about the axis of the inner needle cannula 120, and both the inner needle cannula hub 400 and the outer needle cannula hub 300 are slidably connected to the transition member 253 along the axial direction of the inner needle cannula 120. The transmission member 254 is disposed between the gear set 252 and the transition member 253, and two opposite ends of the transmission member 254 are respectively drivingly connected to the gear set 252 and the transition member 253.

The driving assembly 251 may sequentially drive the transition member 253 to rotate through the gear set 252 and the transmission member 254, thereby enabling the synchronous rotation of the inner needle cannula hub 400 and the outer needle cannula hub 300 around the axis of the inner needle cannula 120.

By adopting the above-described approach, the structure of the transmission assembly can be made relatively compact, thereby reducing the volume of the biopsy needle.

It should be noted that the transition member 253 is provided with a boss 2531 and a stop surface 2533. After the third driving member 910 pushes the outer needle cannula hub 300 and the inner needle cannula hub 400 to impact the transition member 253, both the outer needle cannula hub 300 and the inner needle cannula hub 400 may reach the transition member 253 simultaneously at the same speed. The movement stops when the inner needle cannula hub 400 collides with the boss 2531 of the transition member 253. The inner needle cannula hub 400 and the outer needle cannula hub 300 synchronously move the first stroke along the axial direction of the inner needle cannula 120, completing the initial cutting of the target tissue by the inner needle cannula 120 driven by the inner needle cannula hub 400. The outer needle cannula hub 300 then continues to advance for a certain distance under the action of inertia. The outer needle cannula 130 continues to move the second stroke along the axial direction of the inner needle cannula 120 until it stops upon encountering the stop surface 2533 of the transition member 253. The outer needle cannula hub 300 drives the second elastic sheet 131 of the outer needle cannula 130 to enter the slot of the inner needle cannula 120, thereby completing the cutting of the target tissue.

In one embodiment, the driving assembly 251 includes a pressing assembly 2511 and a sector gear 2512. The pressing assembly 2511 is connected to the sector gear 2512, and operating the pressing assembly 2511 can drive the sector gear 2512 to rotate. The gear set 252 is provided with a first transmission gear 2521 and a second transmission gear 2522. The first transmission gear 2521 is drivingly connected to the sector gear 2512, one end of the transmission member 254 is provided with a third transmission gear 2541, and the second transmission gear 2522 is drivingly connected to the third transmission gear 2541. With such a configuration, the pressing is labor-saving and reliable, and the operation is easy. By driving the sector gear 2512 to rotate via the pressing assembly 2511, the transition member 253 can be sequentially driven to rotate through the gear set 252 and the transmission member 254, thereby enabling the synchronous rotation of the inner needle cannula hub 400 and the outer needle cannula hub 300 around the axis of the inner needle cannula 120. The structure is simple and easy to process.

For example, the first transmission gear 2521 may be a spur gear, and the second transmission gear 2522 may be a bevel gear coaxially arranged with the spur gear. The third transmission gear 2541 may be a bevel gear.

Optionally, the driving assembly 251 includes a rotation axis 2513 connected to the housing 200, and the sector gear 2512 is rotatably connected to the rotation axis 2513. With such a configuration, it is convenient to rotatably dispose the sector gear 2512 on the outside of the housing 200.

In order to make the pressing assembly 2511 and the sector gear 2512 automatically reset after operating the pressing assembly 2511 to drive the sector gear 2512 to rotate, optionally, the pressing assembly 2511 includes a pressing member 5111 and an elastic member 5112 connected to each other. The elastic member 5112 is connected to the sector gear 2512, and the elastic member 5112 is configured to store elastic potential energy when the pressing member 5111 is pressed, so as to drive the pressing member 5111 to reset when the pressing member 5111 is released. In some embodiments, one end of the elastic member 5112 is connected to the sector gear 2512, and the other end is connected to the housing 200. With such a configuration, after operating the pressing assembly 2511 to drive the sector gear 2512 to rotate, the pressing assembly 2511 and the sector gear 2512 may automatically reset.

For example, referring to FIG. 33, when the pressing member 5111 is pressed, the pressing member 5111 drives the sector gear 2512 to rotate around the rotation axis 2513 in a direction indicated by an arrow N. At this time, the elastic member 5112 stores elastic potential energy, and both the inner needle cannula 120 and the outer needle cannula 130 synchronously rotate in the first direction. When the pressing member 5111 is released, the elastic member 5112 drives the pressing member 5111 to reset, and the pressing member 5111 drives the sector gear 2512 to rotate around the rotation axis 2513 in a direction indicated by an arrow M. Both the inner needle cannula 120 and the outer needle cannula 130 synchronously rotate in the second direction. The direction indicated by the arrow N is opposite to the direction indicated by the arrow M, and the second direction is opposite to the first direction. When the pressing member 5111 is released, the elastic member 5112 drives the pressing member 5111 to reset, and the pressing member 5111 can be pressed next time to again drive the sector gear 2512 to rotate around the rotation axis 2513 in the direction indicated by the arrow N. Repeating the above process enables the plurality of synchronous rotations of the inner needle cannula hub 400 and the outer needle cannula hub 300, that is, both the inner needle cannula 120 and the outer needle cannula 130 can be enabled to rotate cyclically between the first direction and the second direction.

It should be noted that, when the pressing member 5111 is released, the elastic member 5112 drives the pressing member 5111 to reset, and the pressing member 5111 drives the sector gear 2512 to rotate around the rotation axis 2513 in the direction indicated by the arrow M. In this state, the sector gear 2512 does not engage with the first transmission gear 2521, and it is an idle stroke. When the pressing member 5111 is pressed, the pressing member 5111 drives the sector gear 2512 to rotate around the rotation axis 2513 in the direction indicated by the arrow N. In this state, the sector gear 2512 engages with the first transmission gear 2521, so as to enable the plurality of synchronous rotations of both the inner needle cannula 120 and the outer needle cannula 130 in the first direction.

The elastic member 5112 may be a spring. The elastic member 5112 may also be a torsion spring, elastic rubber, and the like. Two ends of the elastic member 5112 are respectively connected to the housing 200 and the pressing member 5111.

Optionally, in order to facilitate an operator to operate the pressing assembly 2511, the rotary transmission mechanism is disposed at the distal end of the housing 200. The housing 200 is provided with an opening 201, and at least a portion of the pressing assembly 2511 extends to the outside of the housing 200 through the opening 201. With such a configuration, the operator can drive the sector gear 2512 to rotate by pressing at least a portion of the pressing assembly 2511 that extends to the outside of the housing 200 through the opening 201.

Please refer to FIG. 32 to FIG. 34, in some embodiments, the biopsy needle includes an adjusting member 260. The adjusting member 260 is movably connected to the housing 200. The adjusting member 260 has a button 261 located outside the housing 200. The adjusting member 260 is rotatably connected to the transition member 253 for adjusting the position of the transition member 253 along the axial direction of the inner needle cannula 120.

By adopting the above-described approach, the position of the transition member 253 along the axial direction of the inner needle cannula 120 can be adjusted, and thus the length of the inner needle cannula 120 and the outer needle cannula 130 inserted into the tissue to be cut can be adjusted, that is, the sampling length can be adjusted.

Optionally, the housing 200 is provided with a limiting hole 202. A plurality of limiting portions 203 are disposed in the limiting hole 202, and the plurality of limiting portions 203 are spaced apart along the axial direction of the inner needle cannula 120. The adjusting member 260 includes a matching portion 262, and the matching portion 262 may be selectively engaged with different limiting portions 203 to adjust the position of the transition member 253 along the axial direction of the inner needle cannula 120. With such a configuration, the position of the transition member 253 along the axial direction of the inner needle cannula 120 can be adjusted more conveniently.

The limiting portion 203 may be a slot or a hole, and the matching portion 262 may be a block or a post. Alternatively, the matching portion 262 may be a slot or a hole, and the limiting portion 203 may be a block or a post.

Optionally, the transition member 253 is provided with a sliding groove 2532. One end of the transmission member 254 extends into the sliding groove 2532, and the transmission member 254 may reciprocate within the sliding groove 2532 along the axial direction of the inner needle cannula 120. At least one inner wall surface of the sliding groove 2532 slidably contacts the transmission member 254 to limit the rotation of the transmission member 254 relative to the transition member 253 around the axis of the inner needle cannula 120. With such a configuration, the position of the transition member 253 along the axial direction of the inner needle cannula 120 can be adjusted, and the rotation of the transmission member 254 relative to the transition member 253 around the axis of the inner needle cannula 120 can also be prevented.

For example, the main body of the transmission member 254 is a rod-shaped structure. The third transmission gear 2541 is connected to one end of the rod-shaped structure. The rod-shaped structure has a prismatic rod body, and the prismatic rod body is inserted into the sliding groove 2532 of the transition member 253, such that the transition member 253 can move relative to the prismatic rod body of the transmission member 254, and the transition member 253 cannot rotate relative to the prismatic rod body.

The transition member 253 is provided with the two insertion keys. The two insertion keys are respectively inserted into and cooperate with the holes on the inner needle cannula hub 400 and the outer needle cannula hub 300. Both the inner needle cannula hub 400 and the outer needle cannula hub 300 may move relative to the insertion keys of the transition member 253. In this way, after firing, operating the pressing assembly 2511, its sector gear 2512 may drive the first transmission gear 2521 and the second transmission gear 2522 to rotate. The second transmission gear 2522 then drives the third transmission gear 2541 to rotate. The transmission member 254 drives the transition member 253 to rotate. The transition member 253, in turn, further drives the inner needle cannula hub 400 and the outer needle cannula hub 300 to rotate through its own insertion keys, thereby enabling the function of synchronous rotation of the inner needle cannula 120 and the outer needle cannula 130.

It should be noted that, when the insertion keys are relatively long along the longitudinal direction of the inner needle cannula 120, the inner needle cannula hub 400 and the outer needle cannula hub 300 may move along the insertion keys before the inner needle cannula hub 400 and the outer needle cannula hub 300 are fired to complete sampling.

Please refer to FIG. 32, in some embodiments, the rotary transmission mechanism has a preset transmission ratio, and operation of the driving assembly through the transmission assembly at least enables a rotation angle of the rotary bracket in any rotation direction to be not less than 360°.

By employing a single operation of the rotary transmission mechanism using the above-described approach, the rotation angle of the inner needle cannula 120 and the outer needle cannula 130 in any rotation direction can be not less than 360°, which enables the second elastic sheet 131 of the outer needle cannula 130 to perform a more thorough cutting on the tissue to be cut.

Optionally, a side cutting edge is added to the second elastic sheet 131 of the outer needle cannula 130, and the root portion of the second elastic sheet 131 is strengthened, so as to alleviate the impact received by the second elastic sheet 131 during the rotation process, and reduce the risk of deformation.

For example, the preset transmission ratio of the rotary transmission mechanism may be specifically described as follows.

The transmission ratio of the sector gear 2512 and the first transmission gear 2521 is 1:8, and the transmission ratio of the second transmission gear 2522 and the third transmission gear 2541 is 1:2. That is, when the sector gear 2512 rotates by 1/8 revolution in a single operation, the second transmission gear 2522 rotates by 1/2 revolution, and the third transmission gear 2541 just drives the inner needle cannula hub 400 and the outer needle cannula hub 300 to rotate by 1 full revolution, that is, the rotation angle is 360°.

It is understandable that, when the sector gear 2512 rotates more than 1/8 revolution in a single operation, the rotation angle of the inner needle cannula hub 400 and the outer needle cannula hub 300 in any rotation direction is greater than 360°.

According to spatial conditions, the specific count of teeth is exemplified herein as follows: the sector gear 2512 has 40 teeth, the effective teeth cooperating with the first transmission gear 2521 here are 5 teeth, the first transmission gear 2521 has 10 teeth, the second transmission gear 2522 has 24 teeth, and the third transmission gear 2541 has 12 teeth.

It is understandable that the rotary transmission mechanism employs gear transmission, such that pressing saves effort and is reliable, and operation is easy. Furthermore, it has better precision control for a 360° rotation and a small angle deviation.

Before the biopsy needle provided in the above embodiments is cocked, the second elastic sheet 131 of the outer needle cannula 130 is maintained inserted in the slot of the inner needle cannula 120, and the third driving member 910 is in a relaxed state. Before the biopsy needle is inserted into the human body, it is necessary to perform the cocking. Specifically, the compression of the third driving member 910 can be achieved by pulling a lever 280. When the second latch on the outer needle cannula hub engages with the locking protrusion on the housing 200, the third driving member 910 is fully compressed, and the cocking action is completed.

After the cocking, the core needle 110 is in a protruding state, and the second elastic sheet 131 of the outer needle cannula 130 disengages from the curved slot of the inner needle cannula 120. Next, a gear operation may be performed. The desired gear position for the sampling length may be adjusted by toggling the adjusting member 260. The gear operation may also be performed before cocking. Then, guided by an imaging device, the biopsy needle provided in the embodiments of the present disclosure is inserted into the human body, moved near the target tissue, and after toggling the self-locking key to unlock, the firing and sampling action is performed.

The biopsy needle provided in the embodiments of the present disclosure supports two firing manners, the principles of which are as follows.

In a first manner, for example, in some computed tomography (CT) procedures, an operator can puncture the biopsy needle to a sampling position of the target tissue under the guidance of CT images. Then, by pressing a firing button at the tail end of the biopsy needle, a protruding structure in the firing button pushes away the second latch on the outer needle cannula hub 300, causing it to disengage from the locking protrusion in the housing 200. At this time, the compressed third driving member 910 releases energy, converting the elastic potential energy accumulated during previous compression into kinetic energy, pushing the outer needle cannula hub 300 and the inner needle cannula hub 400 to impinge on the transition member 253. The transition member 253 is provided with a boss 2531 and a stop surface 2533. A small spring is disposed between the outer needle cannula hub 300 and the inner needle cannula hub 400 to maintain a relative movement distance between the outer needle cannula hub 300 and the inner needle cannula hub 400. During the movement process of the outer needle cannula hub 300 and the inner needle cannula hub 400 for firing and sampling, the third driving member 910 pushes the inner needle cannula hub 400, and the inner needle cannula hub 400 pushes the outer needle cannula hub 300 to move from the proximal end to the distal end of the housing 200. Since the elastic force of the third driving member 910 is much higher than the elastic force of the small spring, the outer needle cannula hub 300 and the inner needle cannula hub 400 maintain relative stillness during the movement process for firing, and simultaneously reach the transition member 253 at the same speed. The movement stops when the inner needle cannula hub 400 collides with the boss 2531 of the transition member 253. The inner needle cannula hub 400 and the outer needle cannula hub 300 synchronously move a first stroke along an axial direction of the inner needle cannula 120, thereby completing the initial cutting of the target tissue by the inner needle cannula 120 driven by the inner needle cannula hub 400. Then, the outer needle cannula hub 300 continues to advance a certain distance under the action of inertia. The outer needle cannula 130 continues to move a second stroke along the axial direction of the inner needle cannula 120 until it contacts the stop surface 2533 of the transition member 253 and stops. The outer needle cannula hub 300 drives the second elastic sheet 131 of the outer needle cannula 130 into the slot of the inner needle cannula 120, completing the cutting of the target tissue. At this time, under the action of the small spring and the side latch of the outer needle cannula hub 300, the outer needle cannula hub 300 maintains a staggered state of relative position with the inner needle cannula 120. That is, the second elastic sheet 131 of the outer needle cannula 130 remains inserted in the slot of the inner needle cannula 120, thereby ensuring the reliability of sampling.

Then, the K-th operation of enabling the pressing assembly 2511 to drive the sector gear 2512 to rotate is performed, which sequentially drives the transition member 253 to rotate through the gear set 252 and the transmission member 254, thereby achieving the K-th synchronous rotation of the inner needle cannula hub 400 and the outer needle cannula hub 300, where K is a positive integer greater than or equal to 1.

Next, the (K+1)-th operation of enabling the pressing assembly 2511 to drive the sector gear 2512 to rotate is performed, which sequentially drives the transition member 253 to rotate through the gear set 252 and the transmission member 254, thereby achieving the (K+1)-th synchronous rotation of the inner needle cannula hub 400 and the outer needle cannula hub 300.

The pressing assembly 2511 is repeatedly operated to drive the sector gear 2512 to rotate, until the relatively thorough cutting of the target tissue is performed, so as to obtain the target tissue sample completely separated from the human tissue.

In a second manner, for example, in some ultrasound procedures, an operator can also press the front firing button, which may link with the tail end firing button, thereby repeating the movement process described in the first manner to achieve the firing and sampling of the target tissue.

Then, the K-th operation of enabling the pressing assembly 2511 to drive the sector gear 2512 to rotate is performed, which sequentially drives the transition member 253 to rotate through the gear set 252 and the transmission member 254, thereby achieving the K-th synchronous rotation of the inner needle cannula hub 400 and the outer needle cannula hub 300, where K is a positive integer greater than or equal to 1.

Next, the (K+1)-th operation of enabling the pressing assembly 2511 to drive the sector gear 2512 to rotate is performed, which sequentially drives the transition member 253 to rotate through the gear set 252 and the transmission member 254, thereby achieving the (K+1)-th synchronous rotation of the inner needle cannula hub 400 and the outer needle cannula hub 300.

The pressing assembly 2511 is repeatedly operated to drive the sector gear 2512 to rotate, until the relatively thorough cutting of the target tissue is performed, so as to obtain the target tissue sample completely separated from the human tissue.

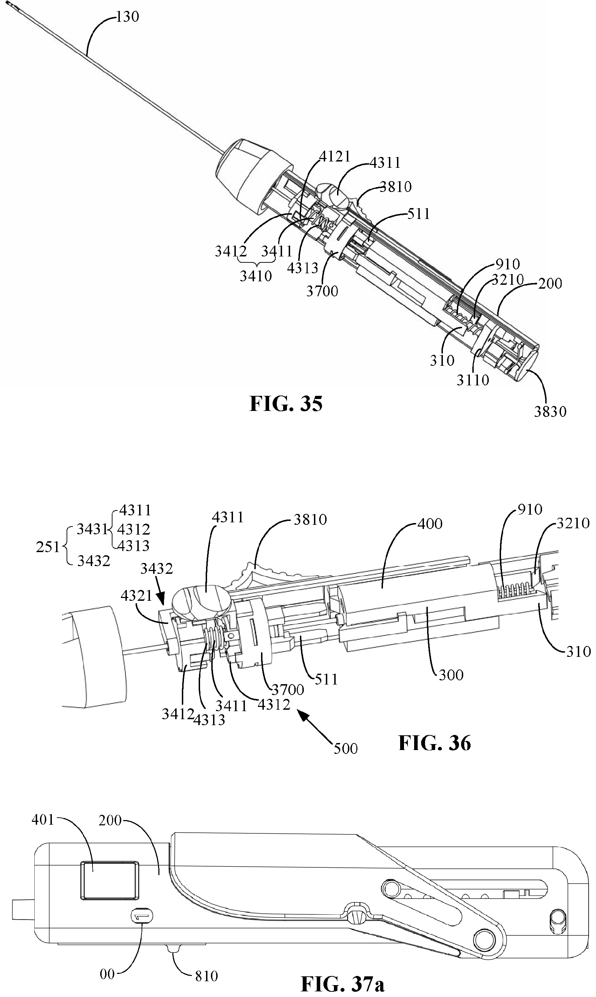

As shown in FIG. 35 and FIG. 36, in one embodiment, a rotary transmission mechanism 500 includes a transmission assembly 3410 and a rotary bracket 510. The transmission assembly 3410 is movably connected to a housing 200. The rotary bracket 510 is rotatably connected to the housing 200. The transmission assembly 3410 is operatively coupled to the rotary bracket 510. An inner needle cannula hub 400 and an outer needle cannula hub 300 are both connectable to the rotary bracket 510. When the transmission assembly 3410 moves relative to the housing 200 along the axis of the housing 200, the transmission assembly 3410 is capable of driving the rotary bracket 510 to rotate relative to the housing 200. The rotary bracket 510 is provided with an avoidance hole extending axially through the rotary bracket 510. The avoidance hole is configured to allow a needle assembly 100 to pass through.

By drivingly connecting the transmission assembly 3410 to the rotary bracket 510, a linear movement of the transmission assembly 3410 along the axis of the housing 200 is converted into a rotary motion of the rotary bracket 510 about the axis of the housing 200. Since the rotary bracket 510 is connectable to the inner needle cannula hub 400 and the outer needle cannula hub 300, when the rotary bracket 510 rotates, the inner needle cannula hub 400 and the outer needle cannula hub 300 can be driven by the rotary bracket 510 to rotate relative to the housing 200, thereby realizing the rotation of an inner needle cannula 120 and an outer needle cannula 130 relative to a core needle 110. Since the inner needle cannula hub 400 and the outer needle cannula hub 300 are disposed at a proximal end of the housing 200, and the transmission assembly 3410 is disposed at a distal end of the housing 200, by providing the avoidance hole on the rotary bracket 510, it facilitates the passage of the inner needle cannula 120 and the outer needle cannula 130, so as to extend out of the housing 200.

In another embodiment, the biopsy needle further includes a fixed ring 3700. The fixed ring 3700 is fixedly connected to the housing 200. An inner peripheral surface of the fixed ring 3700 is provided with a limiting slot extending along a circumferential direction of the fixed ring 3700. An outer periphery of the rotary bracket 510 is disposed within the limiting slot, and the rotary bracket 510 is capable of rotating relative to the fixed ring 3700 along the circumferential direction of the fixed ring 3700.

As shown in FIG. 35 and FIG. 36, in one embodiment, a driving assembly 251 is connected to the housing 200. The driving assembly 251 is operatively movable relative to the housing 200. The driving assembly 251 is connected to the transmission assembly 3410 to drive the transmission assembly 3410 to move relative to the housing 200. By providing the driving assembly 251 and connecting a driving assembly 3430 to the transmission assembly 3410, the driving assembly 251 is enabled to move relative to the housing 200 to drive the transmission assembly 3410 to move relative to the housing 200, which in turn causes the transmission assembly 3410 to drive the rotary bracket 510 to rotate relative to the housing 200.

In one embodiment, the driving assembly 251 includes an elastic component 3431.

The moving member includes a driving screw 3411 threadedly and operatively coupled to the rotary bracket 510.

The two ends of the elastic component 3431 abut respectively against the rotary bracket 510 and the driving screw 3411, so as to enable the driving screw 3411 to move relative to the housing 200.

Specifically, a driving assembly 251 includes an elastic component 3431. The moving member includes a driving screw 3411. The driving screw 3411 is threadedly connected to a rotary bracket 510. A cocking latch 3412 is fixedly connected to the driving screw 3411. When the cocking latch 3412 moves a preset stroke toward a proximal end of the housing 200, the cocking latch 3412 is capable of engaging with the housing 200. The elastic component 3431 is capable of abutting against the cocking latch 3412 to unlock the engaged cocking latch 3412, such that the driving screw 3411 is capable of moving relative to the housing 200.

By threadedly connecting the driving screw 3411 to the rotary bracket 510, since the rotary bracket 510 can only rotate relative to the housing 200, when the driving screw 3411 moves relative to the housing 200, the driving screw 3411 is capable of driving the rotary bracket 510 to rotate relative to the housing 200. By fixedly connecting the cocking latch 3412 to the driving screw 3411, such that when the inner needle cannula 120 and the outer needle cannula 130 are cocked, the cocking latch 3412 is capable of moving along the axis of the housing 200 toward the proximal end of the housing 200 for a certain stroke and then engaging with the housing 200. At this time, the cocking latch 3412 is fixed relative to the housing 200. After the inner needle cannula 120 and the outer needle cannula 130 are fired, the elastic component 3431 moves relative to the housing 200. This movement allows the elastic component 3431 to abut against the cocking latch 3412 and apply a thrust to the cocking latch 3412 in a direction toward a distal end of the housing 200. The thrust thereby unlocks the engagement between the cocking latch 3412 and the housing 200. As a result, the driving screw 3411 is enabled to move relative to the housing 200, thereby driving the rotary bracket 510 to rotate relative to the housing 200.

As shown in FIG. 35 and FIG. 36, in one embodiment, the elastic component 3431 includes a rotary firing button 4311 and a rotary firing latch 4312. The rotary firing button 4311 is partially disposed outside an accommodating chamber. The rotary firing latch 4312 is fixedly connected to the rotary firing button 4311. The rotary firing latch 4312 is capable of abutting against the cocking latch 3412. When the rotary firing button 4311 is operatively moved relative to the housing 200, the rotary firing latch 4312 is capable of applying a force to the cocking latch 3412, so as to disengage the cocking latch 3412 from the housing 200.

By partially disposing the rotary firing button 4311 outside the accommodating chamber, an operator is enabled to apply a force to the rotary firing button 4311. By fixedly connecting the rotary firing latch 4312 to the rotary firing button 4311, such that when the rotary firing button 4311 is operatively moved, the rotary firing latch 4312 is capable of moving with the rotary firing button 4311 relative to the housing 200. Further, the rotary firing latch 4312 is capable of abutting against the cocking latch 3412. When the rotary firing button 4311 is operatively moved relative to the housing 200, the rotary firing latch 4312 is capable of applying the force to the cocking latch 3412, so as to disengage the cocking latch 3412 from the housing 200. Such a configuration is provided to achieve unlocking of the mutual engagement between the cocking latch 3412 and the housing 200.

As shown in FIG. 35 and FIG. 36, in one embodiment, the elastic component 3431 further includes a first elastic member 4313. The first elastic member 4313 is sleeved on the driving screw 3411. The two ends of the first elastic member 4313 abut against the housing 200 and the cocking latch 3412, respectively. When the cocking latch 3412 engages with the housing 200, the first elastic member 4313 is compressed. After the engagement between the cocking latch 3412 and the housing 200 is unlocked, the cocking latch 3412 moves toward the distal end of the housing 200 under the action of the first elastic member 4313.

By arranging the first elastic component 4313 between a housing 200 and a cocking latch 3412, when the cocking latch 3412 is engaged with the housing 200, the first elastic component 4313 is compressed, thereby storing elastic potential energy in the first elastic component 4313. After the engagement of the cocking latch 3412 with the housing 200 is unlocked, the cocking latch 3412 is pushed to move relative to the housing 200 towards a distal end of the housing 200 by the elastic potential energy of the first elastic component 4313. The movement of the cocking latch 3412 drives a driving screw 3411 to move. This further achieves the rotation of a rotary bracket 510 relative to the housing 200.

In the present embodiment, the first elastic component 4313 is a spring. The spring is sleeved on the driving screw 3411. One end of the spring abuts on the cocking latch 3412, and the other end abuts on an end face of a fixing ring 3700. When the cocking latch 3412 moves towards a proximal end of the housing 200, the spring is compressed.

As shown in FIG. 35 and FIG. 36, in one embodiment, a driving assembly 251 further includes a cocking push block 4321. The cocking push block 4321 is movably connected to the housing 200. One end of the cocking push block 4321 abuts on the cocking latch 3412. The cocking push block 4321 is configured to operably drive the cocking latch 3412 to move towards the proximal end of the housing 200 by a preset stroke, thereby causing the cocking latch 3412 to be engaged with the housing 200.

By movably connecting the cocking push block 4321 to the housing 200 and abutting one end of the cocking push block 4321 on the cocking latch 3412, when the cocking push block 4321 is operably moved, the cocking push block 4321 is capable of applying a force on the cocking latch 3412. This force drives the cocking latch 3412 to move towards the proximal end of the housing 200, thereby further achieving the engagement of the cocking latch 3412 with the housing 200. The cocking push block 4321 is mainly used for cocking the cocking latch 3412.

As shown in FIG. 35 and FIG. 36, in one embodiment, the biopsy needle further includes a cocking slider 3810. A portion of the cocking slider 3810 is disposed outside an accommodating chamber, and a portion of the cocking slider 3810 disposed inside the accommodating chamber is configured to abut on the inner needle cannula hub 400 and the outer needle cannula hub 300. The cocking slider 3810 is also connected to the cocking push block 4321 to drive the cocking push block 4321 to move relative to the housing 200. When the cocking slider 3810 is operably moved, the cocking slider 3810 is configured to push the inner needle cannula hub 400 and the outer needle cannula hub 300 to move relative to the housing 200 to a preset distance and be engaged with the housing 200, thereby achieving cocking.

As shown in FIG. 35 to FIG. 36, the cocking slider 3810 is mainly used for cocking the inner needle cannula hub 400 and the outer needle cannula hub 300. Specifically, a part of the cocking slider 3810 is disposed outside the accommodating chamber, to enable an operator to apply a force on the cocking slider 3810, thereby achieving the movement of the cocking slider 3810 relative to the housing 200. Since the portion of the cocking slider 3810 disposed inside the accommodating chamber is configured to abut on the inner needle cannula hub 400 and the outer needle cannula hub 300, when the cocking slider 3810 is operably moved relative to the housing 200, the cocking slider 3810 is configured to push the inner needle cannula hub 400 and the outer needle cannula hub 300 to move relative to the housing 200. This movement causes the inner needle cannula hub 400 and the outer needle cannula hub 300 to be engaged with the housing 200 after moving a certain distance relative to the housing 200, thereby achieving the cocking of the inner needle cannula hub 400 and the outer needle cannula hub 300. Further, the cocking slider 3810 is also connected to the cocking push block 4321, such that when the cocking slider 3810 moves relative to the housing 200, the cocking push block 4321 is simultaneously driven to move relative to the housing 200. This movement achieves the engagement of the cocking latch 3412 with the housing 200, thereby achieving the cocking of the cocking latch 3412.

Specifically, as shown in FIG. 35, the inner needle cannula hub 400 is provided with a third latch 3210, and the outer needle cannula hub 300 is provided with a second latch 310. After the inner needle cannula hub 400 and the outer needle cannula hub 300 move a preset stroke towards the proximal end of the housing 200 under the action of a cocking slider 3810, the third latch 3210 and the second latch 310 can latch with the housing 200.

In one embodiment, as shown in FIG. 35, the biopsy needle further includes a button 3830. The button 3830 is provided at the proximal end of the housing 200. The button 3830 is used to disengage the inner needle cannula hub 400 and the outer needle cannula hub 300 from latching with the housing 200, thereby realizing firing of the inner needle cannula hub 400 and the outer needle cannula 130.

Specifically, as shown in FIG. 35, the proximal end of the housing 200 is provided with an opening communicating with an accommodating chamber of the housing 200. The button 3830 passes through the opening. One end of the button 3830 is located outside the accommodating chamber, and the other end is provided inside the accommodating chamber. Inside the housing 200, a stop block 3110 is provided at a position adjacent to the button 3830 in the accommodating chamber. The ends of the inner needle cannula hub 400 and the outer needle cannula hub 300, close to the proximal end of the housing 200, are both provided with limiting latches. When the inner needle cannula hub 400 and the outer needle cannula hub 300 are pushed by the cocking slider 3810 to move along the proximal end of the housing 200, the latches on the inner needle cannula hub 400 and the outer needle cannula hub 300 may latch with the stop block 3110. It should be noted that a guide rod is also provided on the stop block 3110. A third driving member 910 is sleeved on the outside of the guide rod. One end of the third driving member 910 abuts against the stop block 3110, and the other end abuts against the outer needle cannula hub 300. When the first latch and the second latch 310 on the inner needle cannula hub 400 and the outer needle cannula hub 300 latch with the stop block 3110, the third driving member 910 is in a compressed state.

When it is desired to unlock the inner needle cannula hub 400 and the outer needle cannula hub 300, by pressing the button 3830, the button 3830 moves along the axis of the housing 200 towards the proximal end of the housing 200, thereby causing the button 3830 to abut against the latches of the inner needle cannula hub 400 and the outer needle cannula hub 300, and thereby causing the inner needle cannula hub 400 and the outer needle cannula hub 300 to disengage from the stop block 3110. At this time, the inner needle cannula hub 400 and the outer needle cannula hub 300 move along the axial direction of the housing 200 towards the distal end of the housing 200 under the action of the elastic force of the third driving member 910. This process is the firing process of the inner needle cannula 120 and the outer needle cannula 130. Furthermore, an unlocking plate corresponding to the latches on the inner needle cannula hub 400 and the outer needle cannula hub 300 is provided at the end of the button 3830 located inside the accommodating chamber, so as to apply a force to the latches of the inner needle cannula hub 400 and the outer needle cannula hub 300.

The working principle of the biopsy needle provided by the above embodiments is as described below (with reference to FIG. 35 to FIG. 36).

Before inserting the biopsy needle into the human body, the biopsy needle needs to be cocked. During cocking, the cocking slider 3810 is pushed toward the proximal end of the housing 200. The cocking slider 3810 drives the inner needle cannula hub 400 and the outer needle cannula hub 300 to move along the axis of the housing 200 toward the proximal end of the housing 200. During this process, the third driving member 910 is compressed, thereby accumulating elastic potential energy. This continues until the latching structure in the outer needle cannula hub 300 and the inner needle cannula hub 400 is fixed onto the stop block 3110 within the housing 200, and the third driving member 910 stops compressing. During the process in which the cocking slider 3810 moves toward the proximal end of the housing 200, the cocking slider 3810 drives the cocking push block 4321 to move toward the proximal end of the housing 200. The cocking push block 4321 pushes the cocking latch 3412 to move toward the proximal end of the housing 200. During this process, the cocking latch 3412 compresses the first elastic member 4313 and accumulates the elastic potential energy until the latching structure on the cocking latch 3412 latches with the housing 200. The cocking latch 3412 stops moving, and simultaneously, the first elastic member 4313 stops compressing. At this time, the inner needle cannula hub 400, the outer needle cannula hub 300, and the cocking latch 3412 in the biopsy needle are all in the cocked state. Simultaneously, the inner needle cannula and the outer needle cannula 130, and the rotary transmission mechanism 500 are also in the cocked state.

When the biopsy needle of the present embodiments is inserted into the human body and punctures to a target position of the target tissue under the guidance of an imaging device, by pressing the button 3830 arranged at the proximal end of the housing 200, the button 3830 moves toward the distal end of the housing 200, the unlocking plate on the button 3830 directly abuts against the latching structure in the inner needle cannula hub 400 and the outer needle cannula hub 300, the latching structure is subjected to force and separates from the stopper block 3110. At this time, the elastic potential energy stored in the third driving member 910 during the cocking process is converted into kinetic energy. Since one end of the third driving member 910 is in contact with the outer needle cannula hub 300, during the process of converting the elastic potential energy into kinetic energy, the third driving member 910 pushes the outer needle cannula hub 300 to move along the axial direction of the housing 200 toward the distal end of the housing 200. Simultaneously, the outer needle cannula hub 300 pushes the inner needle cannula hub 400 to move along the axial direction of the housing 200 toward the distal end of the housing 200, thereby driving the inner needle cannula and the outer needle cannula 130 to move toward the distal end of the housing 200.

After the biopsy needle completes firing, the rotary firing button 4311 is toggled, so that the rotary firing button 4311 moves toward the distal end of the housing 200 to drive the rotary firing latch 4312 to move toward the distal end of the housing 200. When the rotary firing latch 4312 moves to abut against the latching structure on the cocking latch 3412, the latching structure of the cocking latch 3412 is subjected to the force of the rotary firing latch 4312 and separates from the housing 200. After the latching structure of the cocking latch 3412 separates from the housing 200, the first elastic member 4313 is no longer compressed. The elastic potential energy stored during the cocking process is converted into kinetic energy, pushing the cocking latch 3412 to move toward the distal end of the housing 200. At this time, the cocking latch 3412 drives the driving screw 3411 to move toward the distal end of the housing 200. Since the rotary bracket 510 cooperates with the driving screw 3411 through a threaded connection, during the process in which the driving screw 3411 moves relative to the housing 200, the rotary bracket 510 rotates with the axis of the housing 200 as a rotation axis under the action of the driving screw 3411. This drives the outer needle cannula hub 300 and the inner needle cannula hub 400 to rotate in the same direction, thereby causing the inner needle cannula and the outer needle cannula 130 to start rotating. During the rotation of the inner needle cannula hub 400, the second elastic sheet 131 extending from the outer needle cannula 130 into the inner needle cannula 120 also rotates, so as to process the sample tissue inside the inner needle cannula by means of the second elastic sheet 131, thereby achieving the purpose of separating the sample tissue from the surrounding tissue.

In some embodiments, FIG. 37a, FIG. 37b, FIG. 38a, and FIG. 38b are schematic diagrams for gear position visualization. FIG. 37a and FIG. 37b show that a current gear position is a first gear position, and FIG. 38a and FIG. 38b show that the current gear position is a third gear position. In practice, more or fewer gear positions than the three gear positions may be set according to actual needs. A gear position window 00 may be opened on a peripheral side surface of the housing 200 to display the current gear position. For example, the gear position window 00 may be disposed near the sampling firing button 401, so as to facilitate confirming the current gear position during a firing operation. Gear position numbers may be pre-marked on the second shifting member. After the gear shift button 810 controls the first shifting member to move a corresponding stroke via the second shifting member, the gear position numbers are visualized through the gear position window 00. Such a configuration eliminates the need to confirm the current gear position by observing the state of the gear shift button 810 by rotating the biopsy needle.

In some embodiments, as shown in FIG. 39, the rotary bracket 510 is rotatably connected to the housing 200 via the bearing 3800. The first driving member 540 is a spring and is sleeved outside the rotary bracket 510. One end of the spring abuts against the moving member, and another end of the spring abuts against an outer ring of the bearing 3800, thereby making the rotation of the rotary bracket 510 and the extension-retraction movement of the spring independent of each other and not interfering with each other.

In some embodiments, as shown in FIG. 39, the first unlocking member 600 only includes a connecting rod 610, and a step is disposed on the connecting rod 610. A protrusion is disposed at the distal end of the outer needle cannula hub 300. When the outer needle cannula hub 300 moves relative to the inner needle cannula hub 400 to a preset distance before abutting against a large end face, the protrusion abuts against the step, thereby pushing the connecting rod 610. When the outer needle cannula hub 300 abuts against the large end face and stops, the protrusion simultaneously pushes the connecting rod 610 to unlock the first latch 522. Therefore, when the biopsy needle of the present embodiment is used, the sampling firing button 401 can achieve a one-key firing function after cocking and puncturing to a sampling position. That is, the sampling firing and the movement for firing the rotary transmission mechanism can be realized by a one-key operation of the sampling firing button 401, without needing to independently configure a firing button for the rotary transmission mechanism.

The respective technical features of the embodiments described above can be arbitrarily combined. For the sake of conciseness of description, not all possible combinations of the various technical features in the above embodiments have been described. However, as long as the combinations of these technical features do not conflict, they should all be considered within the scope of the present disclosure.

The embodiments described above merely express a few embodiments of the present disclosure. Their descriptions are relatively specific and detailed, but they should not be construed as limiting the scope of patent protection of the present disclosure. It should be noted that, for a person having ordinary skill in the art, several variations and improvements may be made without departing from the concept of the present disclosure, and these all fall within the scope of protection of the present disclosure. Therefore, the scope of patent protection of the present disclosure should be subject to the appended claims.

## Claims

1. A biopsy needle, comprising:
an inner needle cannula;
an outer needle cannula sleeved outside the inner needle cannula;
a housing;
an inner needle cannula hub movably disposed in the housing, wherein the inner needle cannula hub is fixedly connected to the inner needle cannula;
an outer needle cannula hub movably disposed in the housing, wherein the outer needle cannula hub is fixedly connected to the outer needle cannula, and the outer needle cannula hub and the inner needle cannula hub are capable of moving relative to each other; and
a rotary transmission mechanism disposed in the housing,
wherein the rotary transmission mechanism is operable to rotate relative to the housing for driving the inner needle cannula and the outer needle cannula to rotate synchronously.

2. The biopsy needle according to claim 1, wherein the rotary transmission mechanism is connected to the inner needle cannula hub and the outer needle cannula hub; and
after the biopsy needle completes a sampling firing operation, the inner needle cannula and the outer needle cannula are driven to rotate synchronously by controlling the rotary transmission mechanism.

3. The biopsy needle according to claim 2, wherein the rotary transmission mechanism includes:
an actuating member rotatably connected to the housing;
a transmission assembly drivingly connected to the actuating member; and
a driving assembly driving the actuating member to rotate via the transmission assembly.

4. The biopsy needle according to claim 3, wherein the actuating member includes a rotary bracket,
the rotary bracket is rotatably disposed relative to the housing,
the outer needle cannula hub and the inner needle cannula hub are movable along an axial direction of the rotary bracket relative to the rotary bracket, and
the rotary bracket is capable of driving the outer needle cannula hub and the inner needle cannula hub to rotate relative to the housing.

5. The biopsy needle according to claim 4, wherein one end of the rotary bracket along an axis of the rotary bracket is configured with an insertion key, and the insertion key is connected to at least one of the outer needle cannula hub or the inner needle cannula hub.

6. The biopsy needle according to claim 5, wherein the transmission assembly includes a moving member, the moving member is movably disposed relative to the housing, the moving member is drivingly connected to the rotary bracket, and the driving assembly drives the rotary bracket to rotate via the moving member.

7. The biopsy needle according to claim 6, wherein a peripheral side surface of the rotary bracket is provided with a driving sliding groove, and the transmission assembly further includes:
a protruding portion connected to the moving member, a portion of the protruding portion being slidably disposed in the driving sliding groove;
wherein when the moving member moves relative to the housing along a first direction, the rotary bracket is driven to rotate around an axis of the housing through the protruding portion sliding along a trajectory of the driving sliding groove.

8. The biopsy needle according to claim 7, wherein the driving assembly includes:
a first driving member, wherein two ends of the first driving member respectively abut against the moving member and the rotary bracket, and the first driving member is capable of providing a driving force for the moving member to move along an axial direction of the housing.

9. The biopsy needle according to claim 7, wherein the driving sliding groove includes a curved groove section, the moving member moves along the first direction, and the protruding portion slides along the curved groove section to drive the rotary bracket to rotate.

10. The biopsy needle according to claim 7, wherein the driving sliding groove includes a linear groove section extending along the axial direction of the rotary bracket, and a curved groove section extending helically around the axial direction of the rotary bracket, and two ends of the curved groove section are correspondingly connected to two ends of the linear groove section.

11. The biopsy needle according to claim 10, wherein each of the linear groove section and the curved groove section includes a head end and a tail end, the head end is an end facing a proximal end of the housing, and the tail end is an end facing a distal end of the housing;
both a head end of the linear groove section and a tail end of the curved groove section are provided with a stop structure, or both a tail end of the linear groove section and a head end of the curved groove section are provided with the stop structure; and
the stop structure is configured to prevent the protruding portion from retreating after the protruding portion passes through the stop structure from one direction.

12. The biopsy needle according to claim 11, wherein the stop structure at the head end of the linear groove section is a stepped structure formed by a groove bottom of the head end of the linear groove section higher than a groove bottom of the head end of the curved groove section, so as to prevent the protruding portion from retreating after the protruding portion moves from the head end of the linear groove section into the curved groove section; and
the stop structure at the tail end of the curved groove section is a stepped structure formed by a groove bottom of the tail end of the curved groove section higher than the tail end of the linear groove section, so as to prevent the protruding portion from retreating after the protruding portion enters the tail end of the linear groove section from the tail end of the curved groove section;
wherein a groove bottom of the curved groove section and a groove bottom of the linear groove section are smooth surfaces.

13. The biopsy needle according to claim 12, wherein the protruding portion includes:
a support base configured with an accommodating chamber having an opening at one end, wherein the support base is fixedly connected to the moving member;
a second driving member disposed in the accommodating chamber, wherein one end of the second driving member abuts against the support base; and
a movable member movably disposed in the accommodating chamber and abutting against another end of the second driving member, wherein a portion of the movable member protrudes from an end face of the support base.

14. The biopsy needle according to claim 11, wherein the stop structure at the head end of the linear groove section is a biasing member, the biasing member is at least partially disposed in the linear groove section;
when the protruding portion moves past the biasing member along a second direction, the biasing member is biased to avoid the protruding portion; and
when the protruding portion moves into the curved groove section, the biasing member resets to block the protruding portion from retreating;
wherein the first direction is opposite to the second direction.

15. The biopsy needle according to claim 6, wherein the driving assembly includes an elastic component, and the moving member includes:
a driving screw threadedly and drivingly connected to the rotary bracket,
wherein two ends of the elastic component respectively abut against the rotary bracket and the driving screw, such that the driving screw is movable relative to the housing.

16. The biopsy needle according to claim 6, wherein the moving member is provided with a first latch, the first latch is latched with the housing after the moving member moves a first preset stroke along an axial direction of the housing toward a proximal end of the housing, and the biopsy needle further includes:
a first unlocking member movably connected to the housing, wherein the first unlocking member is operably connected to the first latch for operably driving the first latch to disengage from the housing.

17. The biopsy needle according to claim 6, wherein the biopsy needle further includes a cocking mechanism, and the cocking mechanism includes:
a cocking trigger rotatably connected to the housing; and
a driving rod movably disposed in the housing and drivingly connected to the cocking trigger, wherein movement of the driving rod along an axis of the housing is driven by rotation of the cocking trigger;
wherein a first stopper and a second stopper spaced apart from each other are configured on the driving rod along an extending direction of the driving rod, the first stopper is for connecting to the moving member, the second stopper is for connecting to the at least one of the outer needle cannula hub or the inner needle cannula hub, and the rotation of the cocking trigger drives the driving rod to move along the axis of the housing, thereby driving the moving member to move as well as driving the at least one of the outer needle cannula hub or the inner needle cannula hub to move.

18. The biopsy needle according to claim 17, wherein the moving member is provided with a stroke avoidance groove, the first stopper is disposed in the stroke avoidance groove, such that the first stopper abuts against the moving member after the driving rod moves a second preset stroke driven by the cocking trigger, thereby driving the moving member to move.

19. The biopsy needle according to claim 4, wherein at least one of the outer needle cannula hub or the inner needle cannula hub is provided with a second latch, and after the at least one of the outer needle cannula hub or the inner needle cannula hub moves a third preset stroke toward a proximal end of the housing, the second latch is latched with the housing.

20. The biopsy needle according to claim 19, wherein a locking slot is disposed on a peripheral side surface of the outer needle cannula hub and/or a peripheral side surface of the inner needle cannula hub, a locking protrusion is disposed on an inner wall of the housing, and when the second latch is latched with the housing, the locking protrusion is engaged in the locking slot.

21. The biopsy needle according to claim 1, wherein the biopsy needle further includes a core needle and a core needle seat, the core needle seat is fixedly connected to a proximal end of the housing, the core needle is fixedly connected to the core needle seat, and an end of the core needle seat facing a distal end of the housing is provided with a first guiding portion; and
an end of the inner needle cannula hub or the outer needle cannula hub facing the proximal end of the housing is provided with a second guiding portion, and when the second latch is latched with the housing, the first guiding portion and the second guiding portion are engaged with each other.

22. The biopsy needle according to any one of claims 5 to 18, wherein the biopsy needle further includes a shifting mechanism, and the shifting mechanism includes:
a first shifting member, wherein the first shifting member is slidably connected to the insertion key for limiting a moving position of the inner needle cannula hub and a moving position of the outer needle cannula hub;
a second shifting member movably disposed in the housing and rotatably connected to the first shifting member, wherein movement of the second shifting member controls a position of the first shifting member relative to the insertion key; and
a gear shift button disposed on the housing and connected to the second shifting member, wherein the gear shift button is operable to move relative to the housing to control the position of the first shifting member through the second shifting member.

23. The biopsy needle according to claim 4, wherein the driving assembly is operable, and the driving assembly is operated after the inner needle cannula hub and the outer needle cannula hub are fired to complete sampling, so as to operably drive the rotary transmission mechanism a plurality of times, thereby enabling a plurality of synchronous rotations of the inner needle cannula hub and the outer needle cannula hub.

24. The biopsy needle according to claim 23, wherein the driving assembly is movably or rotatably disposed on the housing, the transmission assembly includes a gear set, and the driving assembly is drivingly connected to the rotary bracket through the gear set to drive the rotary bracket to rotate.

25. The biopsy needle according to claim 24, wherein the driving assembly includes a pressing assembly and a sector gear, the pressing assembly is connected to the sector gear, and the pressing assembly is pressed to drive the sector gear to rotate; and
the gear set is provided with a first transmission gear and a second transmission gear, the first transmission gear is drivingly connected to the sector gear, one end of the rotary bracket is provided with a third transmission gear, and the second transmission gear is drivingly connected to the third transmission gear.

26. The biopsy needle according to claim 25, wherein the pressing assembly includes a pressing member and an elastic member connected to each other, the pressing member is connected to the sector gear, the elastic member is configured to store elastic potential energy when the pressing member is pressed, so as to drive the pressing member to reset when the pressing member is released; and
the housing is provided with an opening, and at least a portion of the pressing assembly extends to an outside of the housing through the opening.

27. The biopsy needle according to any one of claims 4, wherein the rotary transmission mechanism has a preset transmission ratio, and an operation of the driving assembly through the transmission assembly at least enables a rotation angle of the rotary bracket in any rotation direction to be not less than 360°.
